(19) Europäisches Patentamt
European Patent Office
Office européen des brevets

(11) EP 0 802 198 A2

(12) # EUROPÄISCHE PATENTANMELDUNG

(43) Veröffentlichungstag:
**22.10.1997 Patentblatt 1997/43**

(21) Anmeldenummer: **97105706.2**

(22) Anmeldetag: **07.04.1997**

(51) Int. Cl.$^6$: **C07D 487/04**, C07D 471/14, A61K 31/505, A61K 31/50, A61K 31/495, A61K 31/435 // (C07D487/04, 239:00, 209:00), (C07D487/04, 237:00, 209:00)

(84) Benannte Vertragsstaaten:
**AT BE CH DE DK ES FI FR GB GR IE IT LI LU MC NL PT SE**

(30) Priorität: **18.04.1996 DE 19615265**

(71) Anmelder: **BAYER AG**
**51368 Leverkusen (DE)**

(72) Erfinder:
- **Müller, Ulrich, Dr.**
**42111 Wuppertal (DE)**
- **Eckenberg, Peter, Dr.**
**40699 Erkrath (DE)**
- **Gruetzmann, Rudi, Dr.**
**42657 Solingen (DE)**
- **Bischoff, Hilmar, Dr.**
**42113 Wuppertal (DE)**
- **Denzer, Dirk, Dr.**
**42115 Wuppertal (DE)**
- **Nielsch, Ulrich, Dr.**
**42113 Wuppertal (DE)**

(54) **Neue Pyridazino-, Pyrimido-, Pyrazino- und Triazino-indole**

(57) Die erfindungsgemäßen Pyridazino-, Pyrimido-, Pyrazino- und Triazino-indole werden hergestellt durch Umsetzung der mit den entsprechenden Heterocyclen substituierten Phenylessigsäure-Derivaten gegebenenfalls in einer aktivierten Form mit Phenylglyzinolen. Die heterocyclisch kondensierten Indole eignen sich als Wirkstoffe in Arzneimittel, insbesondere in antiatherosklerotisch wirksamen Arzneimitteln.

EP 0 802 198 A2

**Beschreibung**

Die vorliegende Erfindung betrifft neue Pyridazino-, Pyrimido-, Pyrazino- und Triazino-indole, Verfahren zu ihrer Herstellung und ihre Verwendung als Arzneimittel, insbesondere als antiatherosklerotische Arzneimittel.

Es ist bekannt, daß erhöhte Blutspiegel von Triglyzeriden (Hypertriglyzeridämie) und Cholesterin (Hypercholesterinämie) mit der Genese von atherosklerotischen Gefäßwand-Veränderungen und koronaren Herzkrankheiten assoziiert sind.

Ein deutlich erhöhtes Risiko für die Entwicklung koronarer Herzerkrankungen liegt darüber hinaus vor, wenn diese beiden Risikofaktoren kombiniert auftreten, was wiederum mit einer Überproduktion an Apoliprotein B-100 einhergeht. Es ist daher nach wie vor ein starkes Bedürfnis, wirksame Arzneimittel zur Bekämpfung der Atherosklerose sowie koronarer Herzkrankheiten zur Verfügung zu stellen.

Die vorliegende Erfindung betrifft Pyridazino-, Pyrimido-, Pyrazino- und Triazinoindole der allgemeinen Formel (I)

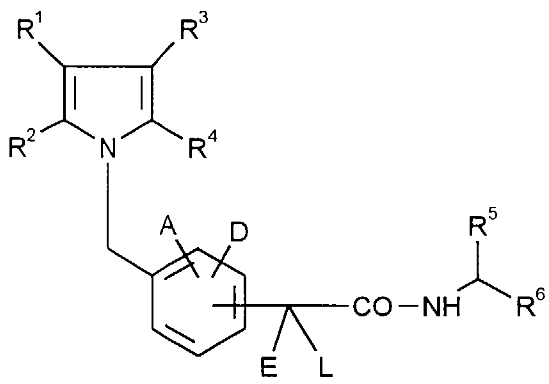

(I)

in welcher

$R^1$ und $R^2$ unter Einbezug der sie verbindenden Doppelbindung gemeinsam einen Phenylring oder einen 5- bis 8-gliedrigen Cycloalken- oder Oxocycloalken-Ring bilden,
der gegebenenfalls bis zu 3-fach gleich oder verschieden durch Halogen, Trifluormethyl, Carboxy, Hydroxy, durch geradkettiges oder verzweigtes Alkoxy oder Alkoxycarbonyl mit jeweils bis zu 6 Kohlenstoffatomen oder durch geradkettiges oder verzweigtes Alkyl mit bis zu 6 Kohlenstoffatomen substituiert ist, das seinerseits durch Hydroxy oder durch geradkettiges oder verzweigtes Alkoxy mit bis zu 4 Kohlenstoffatomen substituiert sein kann,

$R^3$ und $R^4$ unter Einbezug der Doppelbindung gemeinsam einen Rest der Formel

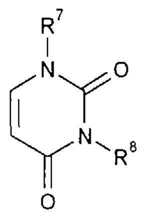 , 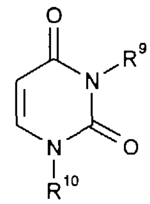 , 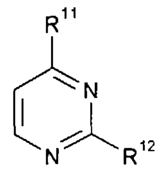 , 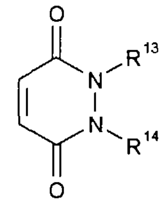 ,

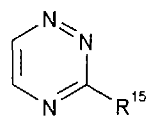 oder 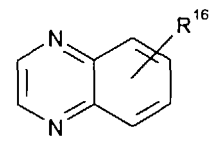

bilden,
worin

$R^7$, $R^8$, $R^9$, $R^{10}$, $R^{11}$, $R^{12}$, $R^{13}$, $R^{14}$, $R^{15}$ und $R^{16}$ gleich oder verschieden sind und Wasserstoff Carboxyl, geradkettiges oder verzweigtes Alkoxy, Alkylthio, Acyl oder Alkoxycarbonyl mit jeweils bis zu 6 Kohlenstoffatomen oder geradkettiges oder verzweigtes Alkyl mit bis zu 6 Kohlenstoffatomen bedeuten, das gegebenenfalls durch Hydroxy substituiert ist,

oder

$R^1$ und $R^2$ unter Einbezug der Doppelbindung einen Pyridylring bilden, und

$R^3$ und $R^4$ ebenfalls unter Einbezug der Doppelbindung gemeinsam einen Pyridylring bilden, wobei beide Pyridylringe gegebenenfalls bis zu 3-fach gleich oder verschieden durch Halogen, Trifluormethyl, Carboxy, Hydroxy, durch geradkettiges oder verzweigtes Alkoxy oder Alkoxycarbonyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy oder durch geradkettiges oder verzweigtes Alkoxy mit bis zu 4 Kohlenstoffatomen substituiert ist,

A und D gleich oder verschieden sind und für Wasserstoff, Halogen, Trifluormethyl, Hydroxy oder geradkettiges oder verzweigtes Alkyl oder Alkoxy mit jeweils bis zu 5 Kohlenstoffatomen stehen,

E und L gleich oder verschieden sind und
für Wasserstoff, Cycloalkyl mit 3 bis 8 Kohlenstoffatomen oder geradkettiges oder verzweigtes Alkyl mit bis zu 10 Kohlenstoffatomen stehen, das gegebenenfalls durch Cycloalkyl mit 3 bis 6 Kohlenstoffatomen substituiert ist, oder
für Phenyl stehen, das gegebenenfalls durch Halogen oder Trifluormethyl substituiert ist, oder

E und L gemeinsam mit dem Kohlenstoffatom einen 4-8 gliedrigen Cycloalkylring bilden,

$R^5$ für Phenyl oder für einen 5- bis 7-gliedrigen gesättigten oder ungesättigten Heterocyclus mit bis zu 3 Heteroatomen aus der Reihe S, N und/oder O steht,
wobei die Cyclen gegebenenfalls bis zu 3-fach gleich oder verschieden durch Nitro, Carboxy, Halogen, Cyano oder durch geradkettiges oder verzweigtes Alkenyl oder Alkoxycarbonyl mit jeweils bis zu 6 Kohlenstoffatomen oder durch geradkettiges oder verzweigtes Alkyl mit bis zu 6 Kohlenstoffatomen substituiert sind, das gegebenenfalls durch Hydroxy, Carboxy oder durch geradkettiges oder verzweigtes Alkoxy oder Alkoxycarbonyl mit jeweils bis zu 6 Kohlenstoffatomen substituiert ist,
und/oder die Cyclen gegebenenfalls durch eine Gruppe der Formel $-OR^{17}$ oder $-NR^{18}R^{19}$ substituiert sind,

worin

$R^{17}$ Wasserstoff oder geradkettiges oder verzweigtes Alkyl oder Alkenyl mit jeweils bis zu 6 Kohlenstoffatomen bedeutet,

$R^{18}$ bzw. $R^{19}$ gleich oder verschieden sind und Phenyl, Wasserstoff oder geradkettiges oder verzweigtes Alkyl mit bis zu 6 Kohlenstoffatomen bedeuten
oder geradkettiges oder verzweigtes Acyl mit bis zu 8 Kohlenstoffatomen bedeuten, das gegebenenfalls durch eine Gruppe der Formel $-NR^{20}R^{21}$ substituiert ist,
worin

$R^{20}$ und $R^{21}$ gleich oder verschieden sind und Wasserstoff oder geradkettiges oder verzweigtes Acyl mit bis zu 8 Kohlenstoffatomen bedeuten,

$R^6$ für Wasserstoff, Carboxy oder für geradkettiges oder verzweigtes Alkoxycarbonyl mit bis zu 5 Kohlenstoffatomen steht,
oder für geradkettiges oder verzweigtes Alkyl mit bis zu 6 Kohlenstoffatomen steht, das gegebenenfalls durch Hydroxy oder durch eine Gruppe der Formel $-O-CO-R^{22}$ substituiert ist,
worin

$R^{22}$ Phenyl bedeutet, das gegebenenfalls bis zu 3-fach gleich oder verschieden durch Halogen, Hydroxy oder durch geradkettiges oder verzweigtes Alkyl mit bis zu 5 Kohlenstoffatomen substituiert ist, oder geradkettiges oder verzweigtes Alkyl oder Alkenyl mit jeweils bis zu 22 Kohlenstoffatomen bedeutet, die gegebenenfalls durch eine Gruppe der Formel $-OR^{23}$ substituiert sind,
worin

$R^{23}$ Wasserstoff, Benzyl, Triphenylmethyl oder geradkettiges oder verzweigtes Acyl mit bis zu 6 Kohlenstoffatomen bedeutet,

gegebenenfalls in einer isomeren Form und deren Salze.

Die erfindungsgemäßen Pyridazino-, Pyrimido-, Pyrazino- und Triazino-indole können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen, welche eine freie Carboxylgruppe besitzen, sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Der Cycloalken-Rest ($R^1/R^2$) steht unter Einbezug der Doppelbindung des Grundgerüstes im Rahmen der Erfindung im allgemeinen für einen 5- bis 8-gliedrigen, vorzugsweise 5- bis 7-gliedrigen Kohlenwasserstoffrest wie beispielsweise für einen Cyclobuten-, Cyclopenten-, Cyclohexen- oder Cyclohepten-Rest. Bevorzugt sind der Cyclopenten-, Cyclohexen-, Cycloocten- und Cyclohepten-Rest.

Heterocyclus steht im Rahmen der Erfindung im allgemeinen für einen gesättigten oder ungesättigten 5- bis 7-gliedrigen, vorzugsweise 5- bis 6-gliedrigen Heterocyclus der bis zu 3 Heteroatome aus der Reihe S, N und/oder O enthalten kann. Beispielsweise seien genannt: Pyridyl, Thienyl, Furyl, Pyrrolyl, Thiazolyl, Oxazolyl, Imidazolyl, Morpholinyl oder Piperidyl. Bevorzugt sind Pyridyl und Thienyl.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die

Enantiomeren als auch Diastereomeren oder deren jeweiligen Mischungen. Diese Mischungen der Enantiomeren und Diastereomeren lassen sich in bekannter Weise in die stereoisomer einheitlichen Bestandteile trennen.

Bevorzugt sind Verbindungen der allgemeinen Formel (I),
in welcher

$R^1$ und $R^2$ unter Einbezug der sie verbindenden Doppelbindung gemeinsam einen Phenylring oder einen Cyclopenten-, Cyclohexen-, Cyclohepten-, Cycloocten-, Oxocyclopenten-, Oxocyclohexen-, Oxocyclohepten- oder Oxocycloocten-Rest bilden,
der gegebenenfalls bis zu 2-fach gleich oder verschieden durch Fluor, Chlor, Brom, Trifluormethyl, Carboxy, Hydroxy, durch geradkettiges oder verzweigtes Alkoxy oder Alkoxycarbonyl mit jeweils bis zu 4 Kohlenstoffatomen oder durch geradkettiges oder verzweigtes Alkyl mit bis zu 4 Kohlenstoffatomen substituiert ist, das seinerseits durch Hydroxy oder durch geradkettiges oder verzweigtes Alkoxy mit bis zu 3 Kohlenstoffatomen substituiert sein kann,

$R^3$ und $R^4$ unter Einbezug der Doppelbindung gemeinsam einen Rest der Formel

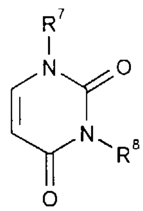 , 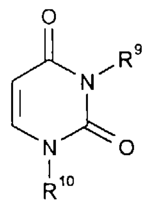 , 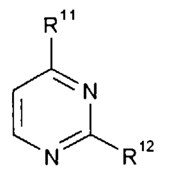 , 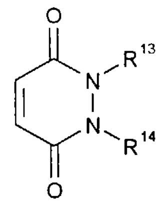 ,

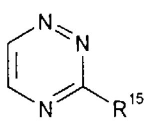 oder 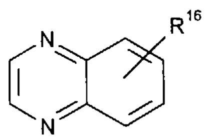

bilden,
worin

$R^7$, $R^8$, $R^9$, $R^{10}$, $R^{11}$, $R^{12}$, $R^{13}$, $R^{14}$, $R^{15}$ und $R^{16}$ gleich oder verschieden sind und Wasserstoff, geradkettiges oder verzweigtes Alkoxy, Alkylthio, Acyl oder Alkoxycarbonyl mit jeweils bis zu 4 Kohlenstoffatomen oder geradkettiges oder verzweigtes Alkyl mit bis zu 4 Kohlenstoffatomen bedeuten, das gegebenenfalls durch Hydroxy substituiert ist,

oder

$R^1$ und $R^2$ unter Einbezug der Doppelbindung einen Pyridylring bilden, und

$R^3$ und $R^4$ ebenfalls unter Einbezug der Doppelbindung gemeinsam einen Pyridylring bilden, wobei beide Pyridylringe gegebenenfalls bis zu 2-fach gleich oder verschieden durch Fluor, Chlor, Brom, Trifluormethyl, Carboxy, Hydroxy, durch geradkettiges oder verzweigtes Alkoxy oder Alkoxycarbonyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy oder durch geradkettiges oder verzweigtes Alkoxy mit bis zu 3 Kohlenstoffatomen substituiert ist,

A und D gleich oder verschieden sind und
für Wasserstoff, Fluor, Chlor, Brom, Trifluormethyl, Hydroxy oder geradkettiges oder verzweigtes Alkyl oder Alkoxy mit jeweils bis zu 4 Kohlenstoffatomen stehen,

E und L gleich oder verschieden sind und
für Wasserstoff, Cyclopropyl, Cyclobutyl, Cyclopentyl, Cyclohexyl, Cycloheptyl, Cyclooctyl oder für geradkettiges oder verzweigtes Alkyl mit bis zu 8 Kohlenstoffatomen stehen, das gegebenenfalls durch

Cyclopropyl, Cyclopentyl oder Cyclohexyl substituiert ist, oder
für Phenyl stehen, das gegebenenfalls durch Fluor, Chlor oder Brom substituiert ist, oder

E und L gemeinsam mit dem Kohlenstoffatom einen 4-7 gliedrigen Cycloalkylring bilden,

$R^5$ für Phenyl, Pyridyl, Furyl, Thienyl oder Imidazolyl steht, die gegebenenfalls bis zu 2-fach gleich oder verschieden durch Nitro, Carboxy, Fluor, Chlor, Brom, Cyano, durch geradkettiges oder verzweigtes Alkenyl oder Alkoxycarbonyl mit jeweils bis zu 4 Kohlenstoffatomen oder durch geradkettiges oder verzweigtes Alkyl mit bis zu 5 Kohlenstoffatomen substituiert sind, das gegebenenfalls durch Hydroxy, Carboxy oder durch geradkettiges oder verzweigtes Alkoxy oder Alkoxycarbonyl mit jeweils bis zu 5 Kohlenstoffatomen substituiert ist,
und/oder die Cyclen gegebenenfalls durch eine Gruppe der Formel $-OR^{17}$ oder $-NR^{18}R^{19}$ substituiert sind,
worin

$R^{17}$ Wasserstoff oder geradkettiges oder verzweigtes Alkyl oder Alkenyl mit jeweils bis zu 4 Kohlenstoffatomen bedeutet,

$R^{18}$ und $R^{19}$ gleich oder verschieden sind und Phenyl, Wasserstoff oder geradkettiges oder verzweigtes Alkyl mit bis zu 5 Kohlenstoffatomen bedeuten,
oder geradkettiges oder verzweigtes Acyl mit bis zu 6 Kohlenstoffatomen bedeuten, das gegebenenfalls durch eine Gruppe der Formel $-NR^{20}R^{21}$ subsituiert ist,
worin

$R^{20}$ und $R^{21}$ gleich oder verschieden sind und Wasserstoff oder geradkettiges oder verzweigtes Acyl mit bis zu 6 Kohlenstoffatomen bedeuten,

$R^6$ für Wasserstoff Carboxy oder für geradkettiges oder verzweigtes Alkoxycarbonyl mit bis zu 4 Kohlenstoffatomen steht,
oder für geradkettiges oder verzweigtes Alkyl mit bis zu 5 Kohlenstoffatomen steht, das gegebenenfalls durch Hydroxyl oder durch eine Gruppe der Formel $-O-CO-R^{22}$ substituiert ist,
worin

$R^{22}$ Phenyl bedeutet, das gegebenenfalls bis zu 3-fach gleich oder verschieden durch Fluor, Chlor, Brom, Hydroxy oder durch geradkettiges oder verzweigtes Alkyl bis zu 4 Kohlenstoffatomen substituiert ist, oder geradkettiges oder verzweigtes Alkyl oder Alkenyl mit jeweils bis zu 20 Kohlenstoffatomen bedeutet, die gegebenenfalls durch eine Gruppe der Formel $-OR^{23}$ substituiert sind,
worin

$R^{23}$ Wasserstoff, Benzyl, Triphenylmethyl oder geradkettiges oder verzweigtes Acyl mit bis zu 5 Kohlenstoffatomen bedeutet,

gegebenenfalls in einer isomeren Form und deren Salze.

Besonders bevorzugt sind Verbindungen der allgemeinen Formel (I),
in welcher

$R^1$ und $R^2$ unter Einbezug der sie verbindenden Doppelbindung gemeinsam einen Phenylring oder einen Cyclopenten-, Cyclohexen-, Cyclohepten-, Cycloocten-, Oxocyclopenten-, Oxocyclohexen-, Oxocyclohepten- oder Oxocycloocten-Rest bilden,
der gegebenenfalls bis zu 2-fach gleich oder verschieden durch Fluor, Chlor, Brom, Trifluormethyl, Carboxy, Hydroxy, durch geradkettiges oder verzweigtes Alkoxy oder Alkoxycarbonyl mit jeweils bis zu 3 Kohlenstoffatomen oder durch geradkettiges oder verzweigtes Alkyl mit bis zu 3 Kohlenstoffatomen substituiert ist, das seinerseits durch Hydroxy, Methoxy oder Ethoxy substituiert sein kann,

$R^3$ und $R^4$ unter Einbezug der Doppelbindung gemeinsam einen Rest der Formel

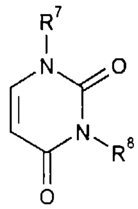 , 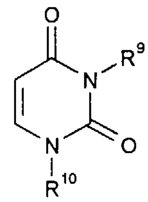 , 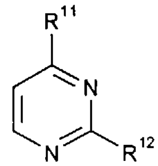 , 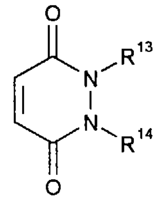 ,

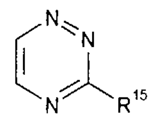 oder 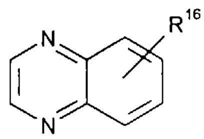

bilden,
worin

$R^7$, $R^8$, $R^9$, $R^{10}$, $R^{11}$, $R^{12}$, $R^{13}$, $R^{14}$, $R^{15}$ und $R^{16}$ gleich oder verschieden sind und Wasserstoff, geradkettiges oder verzweigtes Alkoxy oder Alkylthio mit jeweils bis zu 3 Kohlenstoffatomen oder geradkettiges oder verzweigtes Alkyl mit bis zu 3 Kohlenstoffatomen bedeuten, das gegebenenfalls durch Hydroxy substituiert ist,

oder

$R^1$ und $R^2$ unter Einbezug der Doppelbindung einen Pyridylring bilden, und

$R^3$ und $R^4$ ebenfalls unter Einbezug der Doppelbindung gemeinsam einen Pyridylring bilden, wobei beide Pyridylringe gegebenenfalls bis zu 2-fach gleich oder verschieden durch Fluor, Chlor, Brom, Trifluormethyl, Carboxy, Hydroxy, durch geradkettiges oder verzweigtes Alkoxy oder Alkoxycarbonyl mit jeweils bis zu 3 Kohlenstoffatomen oder durch geradkettiges oder verzweigtes Alkyl mit bis zu 3 Kohlenstoffatomen substituiert sind, das seinerseits durch Hydroxy, Methoxy oder Ethoxy substituiert ist,

A und D gleich oder verschieden sind und
für Wasserstoff, Fluor, Chlor, Brom oder Trifluormethyl stehen,

E und L gleich oder verschieden sind und
für Wasserstoff, Cyclopropyl, Cyclobutyl, Cyclopentyl, Cyclohexyl, Cycloheptyl, Cyclooctyl oder für geradkettiges oder verzweigtes Alkyl mit bis zu 6 Kohlenstoffatomen stehen, das gegebenenfalls durch Cyclopentyl oder Cyclohexyl substituiert ist, oder
für Phenyl stehen, das gegebenenfalls durch Fluor, Chlor oder Brom substituiert ist, oder

E und L gemeinsam mit dem Kohlenstoffatom einen 5-7 gliedrigen Cycloalkylring bilden,

$R^5$ für Phenyl, Pyridyl oder Thienyl steht, die gegebenenfalls bis zu 2-fach gleich oder verschieden durch Nitro, Carboxy, Fluor, Chlor, Brom, Cyano, durch geradkettiges oder verzweigtes Alkenyl oder Alkoxycarbonyl mit jeweils bis zu 3 Kohlenstoffatomen oder durch geradkettiges oder verzweigtes Alkyl mit bis zu 4 Kohlenstoffatomen substituiert sind, das gegebenenfalls durch Hydroxy, Carboxy oder durch geradkettiges oder verzweigtes Alkoxy oder Alkoxycarbonyl mit jeweils bis zu 4 Kohlenstoffatomen substituiert ist,
und/oder die Cyclen gegebenenfalls durch eine Gruppe der Formel $-OR^{17}$ oder $-NR^{18}R^{19}$ substituiert sind,
worin

$R^{17}$ Wasserstoff oder geradkettiges oder verzweigtes Alkyl oder Alkenyl mit jeweils bis zu 3 Kohlenstoffatomen bedeutet,

$R^{18}$ und $R^{19}$ gleich oder verschieden sind und Phenyl, Wasserstoff oder geradkettiges oder verzweigtes Alkyl mit bis zu 4 Kohlenstoffatomen bedeuten, oder geradkettiges oder verzweigtes Acyl mit bis zu 5 Kohlenstoffatomen bedeuten, das gegebenenfalls durch eine Gruppe der Formel $-NR^{20}R^{21}$ substituiert ist,
worin

$R^{19}$ und $R^{20}$ gleich oder verschieden sind und Wasserstoff oder geradkettiges oder verzweigtes Acyl mit bis zu 5 Kohlenstoffatomen bedeuten,

$R^6$ für Wasserstoff, Carboxy oder für geradkettiges oder verzweigtes Alkoxycarbonyl mit bis zu 3 Kohlenstoffatomen steht, oder für geradkettiges oder verzweigtes Alkyl mit bis zu 4 Kohlenstoffatomen steht, das gegebenenfalls durch Hydroxyl oder durch eine Gruppe der Formel $-O-CO-R^{22}$ substituiert ist,
worin

$R^{22}$ Phenyl bedeutet, das gegebenenfalls bis zu 3-fach gleich oder verschieden durch geradkettiges oder verzweigtes Alkyl mit bis zu 3 Kohlenstoffatomen substituiert ist, oder geradkettiges oder verzweigtes Alkyl oder Alkenyl mit jeweils bis zu 19 Kohlenstoffatomen bedeuten, die gegebenenfalls durch eine Gruppe der Formel $-OR^{23}$ substituiert sind,
worin

$R^{23}$ Wasserstoff, Benzyl, Triphenylmethyl oder geradkettiges oder verzweigtes Acyl mit bis zu 4 Kohlenstoffatomen bedeutet,

gegebenenfalls in einer isomeren Fom und deren Salze.

Ganz besonders bevorzugt sind Verbindungen der allgemeinen Formel (I), in welchen

A und D für Wasserstoff stehen.

Außerdem wurde ein Verfahren zur Herstellung der erfindungsgemäßen Verbindungen der allgemeinen Formel (I), gefunden, dadurch gekennzeichnet, daß man racemische oder auch bereits enantiomerenreine Carbonsäuren oder deren aktivierte Derivate der allgemeinen Formel (II)

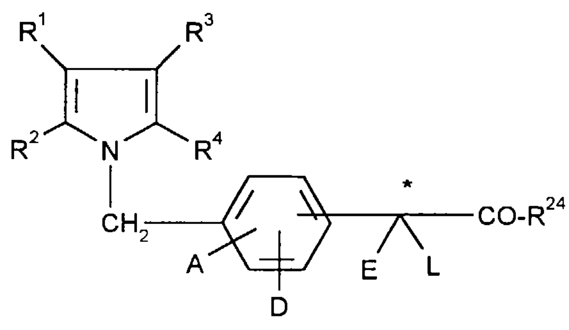

(II)

in welcher

A, D, E, L, $R^1$, $R^2$, $R^3$ und $R^4$ die oben angegebene Bedeutung haben,
und

$R^{24}$ für Hydroxy oder für einen aktivierenden Rest, vorzugsweise Chlorid steht,

mit Verbindungen der allgemeinen Formel (III)

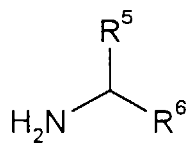 (III)

in welcher

$R^5$ und $R^6$ die oben angegebene Bedeutung haben,

in inerten Lösemitteln, gegebenenfalls in Anwesenheit von Basen und/oder Hilfsstoffen amidiert.

Das erfindungsgemäße Verfahren kann durch folgendes Formelschema beispielhaft erläutert werden:

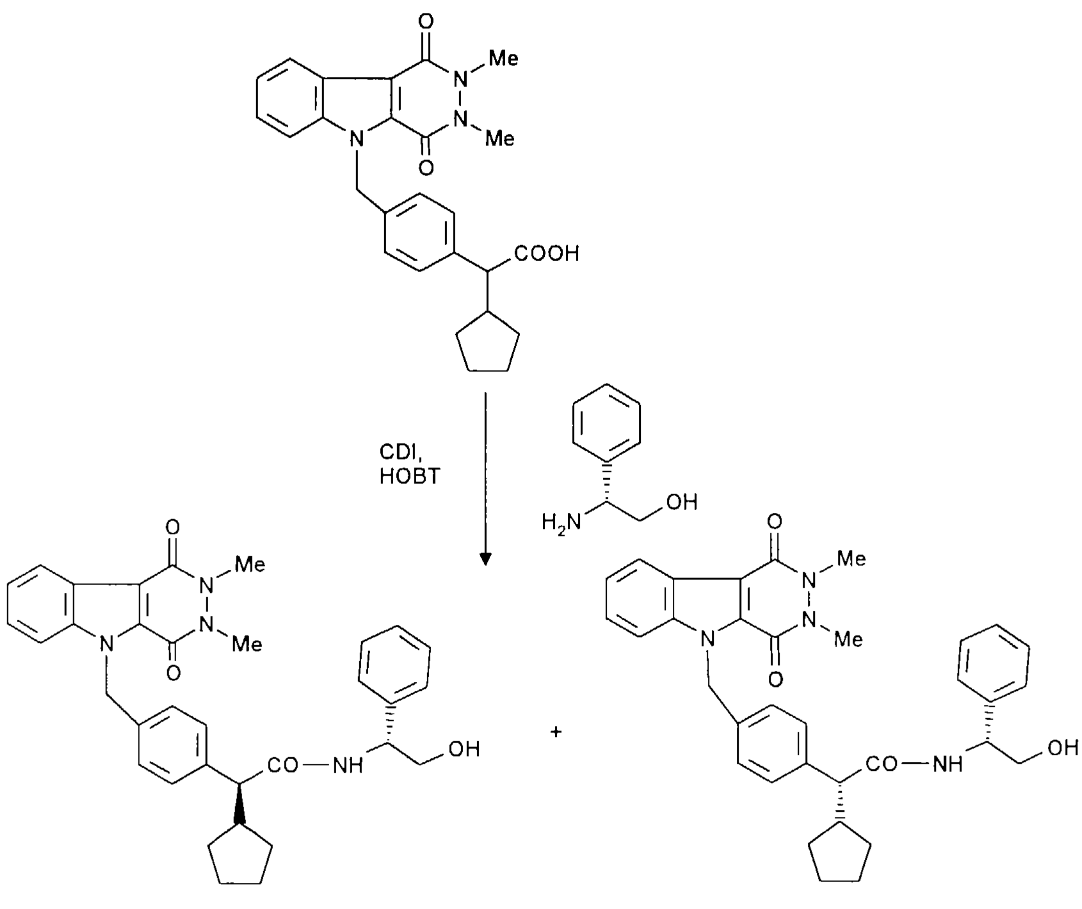


Als Lösemittel für die Amidierung eignen sich hierbei inerte organische Lösemittel, die sich unter den Reaktionsbedingungen nicht verändern. Hierzu gehören Ether, wie Diethylether oder Tetrahydrofuran, Halogenkohlenwasserstoffe wie Dichlormethan, Trichlormethan, Tetrachlormethan, 1,2-Dichlorethan, Trichlorethan, Tetrachlorethan, 1,2-Dichlorethylen oder Trichlorethylen, Kohenwasserstoffe wie Benzol, Xylol, Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt sind Dichlormethan, Tetrahydrofuran, Aceton und Dimethylformamid.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium -oder Kaliumethanolat oder Kalium-tert.butylat, oder organische Amine (Trialkyl-($C_1$-$C_6$)amine) wie Triethyl-

amin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle wie Natrium und deren Hydride wie Natriumhydrid einzusetzen. Bevorzugt sind Natrium- und Kaliumcarbonat und Triethylamin.

Die Base wird in einer Menge von 1 mol bis 5 mol, bevorzugt von 1 mol bis 3 mol, bezogen auf 1 mol der Verbindung der allgemeinen Formel (II) eingesetzt.

Die Reaktion wird im allgemeinen in einem Temperaturbereich von 0°C bis 150°C, bevorzugt von +20°C bis +110°C durchgeführt.

Die Umsetzung kann bei normalem, erhöhtem oder bei erniedrigtem Druck durchgeführt werden (z.B. 0,5 bis 5 bar). Im allgemeinen arbeitet man bei Normaldruck.

Die Umsetzung kann gegebenenfalls auch über die aktivierte Stufe der Säurehalogenide, die aus den entsprechenden Säuren durch Umsetzung mit Thionylchlorid, Phosphortrichlorid, Phosphorpentachlorid, Phosphortribromid oder Oxalylchlorid hergestellt werden können, verlaufen.

Die oben aufgeführten Basen können auch als säurebindende Hilfsmittel für die Amidierung eingesetzt werden.

Als Hilfsstoffe eignen sich ebenso Dehydratisierungsreagenzien. Dazu gehören beispielsweise Carbodiimide wie Diisopropylcarbodiimid, Dicyclohexylcarbodiimid oder N-(3-Dimethylaminopropyl)-N'ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Iso-butylchloroformat oder Benzotriazolyloxy-tris-(dimethylamino)phosphoniumhexa-fluorophosphat oder Phosphorsäurediphenyl-esteramid oder Methan-sulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die Hilfsstoffe werden im allgemeinen in einer Menge von 0,5 bis 3 mol, bevorzugt von 1 bis 1,5 mol, bezogen auf 1 mol der entsprechenden Carbonsäuren, eingesetzt.

Die Carbonsäuren der allgemeinen Formel (II) können hergestellt werden, indem man zunächst durch Umsetzung von Verbindungen der allgemeinen Formel (IV)

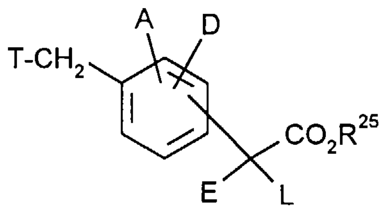

(IV)

in welcher

| | |
|---|---|
| A, D, E und L | die oben angegebene Bedeutung haben, |
| T | für eine typische Abgangsgruppe wie beispielsweise Chlor, Brom, Jod, Tosylat oder Mesylat, vorzugsweise für Brom steht, und |
| $R^{25}$ | für $(C_1\text{-}C_4)$-Alkyl steht, |

mit Verbindungen der allgemeinen Formel (V)

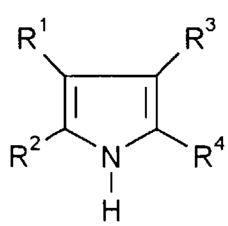

(V)

in welcher

$R^1$, $R^2$, $R^3$ und $R^4$ die oben angegebene Bedeutung haben,

die Verbindungen der allgemeinen Formel (VI)

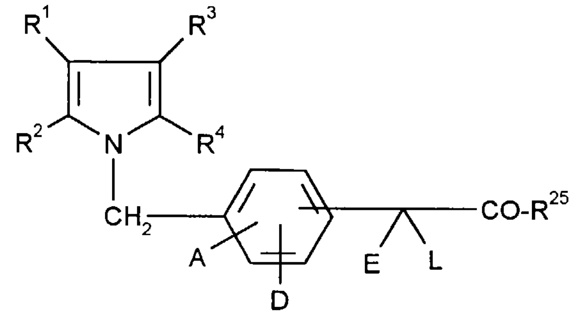

(VI)

in welcher

A, D, E, L, $R^1$, $R^2$, $R^3$, $R^4$ und $R^{25}$ die oben angegebene Bedeutung haben,

in inerten Lösemitteln, gegebenenfalls in Anwesenheit einer Base hergestellt, und anschließend die Ester nach üblichen Methoden verseift.

Die enantiomerenreinen Säuren, d.h. Verbindungen der Formel (II), bei denen E und L verschieden sein müssen, erhält man darüber hinaus ausgehend von den D -oder L-Menthyl-Estern der allgemeinen Formel (VII)

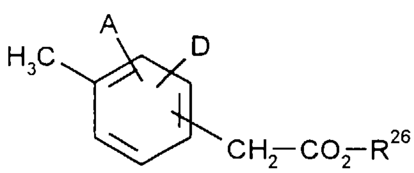

(VII)

in welcher

A und D die oben angegebene Bedeutung haben und

$R^{26}$ für D- oder L-Menthyl steht,

durch Umsetzung mit Verbindungen der allgemeinen Formeln (VIIIa) und (VIIIb)

E-Z (VIIIa)

L-Z (VIIIb)

in welcher

E bzw. L verschieden sind und ansonsten die angegebene Bedeutung haben, und

Z für Halogen, vorzugsweise Brom steht,

die enantiomerenreinen Menthyl-Ester der allgemeinen Formel (IXa) und (IXb)

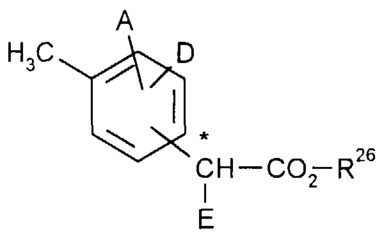 (IXa) 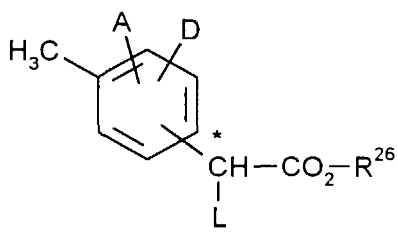 (IXb)

in welcher

A, D, E, L und $R^{26}$ die angegebene Bedeutung haben,

herstellt,
diese in einem nächsten Schritt durch eine Halogenierung in die Verbindungen der allgemeinen Formeln (Xa) und (Xb)

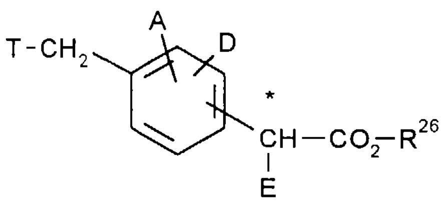 (Xa)

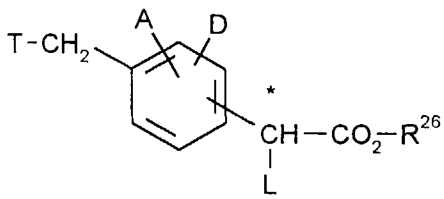 (Xb)

in welcher

A, D, E, L, T und $R^{26}$ die angegebene Bedeutung haben

überführt,
anschließend durch Umsetzung mit den Verbindungen der allgemeinen Formel (V) die enantiomerenreinen Verbindungen der allgemeinen Formeln (XIa) und (XIb)

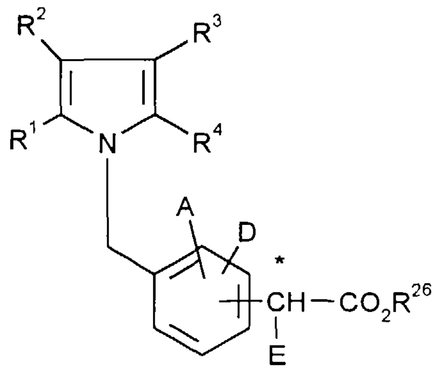

(XIa)

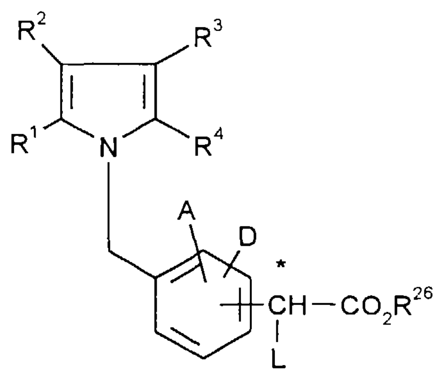

(XIb)

in welcher

A, D, E, L, $R^1$, $R^2$, $R^3$, $R^4$ und $R^{26}$ die angegebene Bedeutung haben,

herstellt,
und diese dann durch Hydrolyse in die enantiomerenreinen Säuen der allgemeinen Formel (II) und gegebenenfalls durch Umsetzung mit aktivierenden Reagenzien in die entsprechenden aktivierten Carbonsäurederivate der allgemeinen Formel (II) überführt.

Außerdem können die enantiomerenreinen Säuren der Formel (II) hergestellt werden, indem man zunächst racemische Carbonsäuren der allgemeinen Formel (XII)

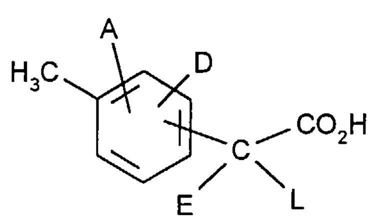

(XII)

in welcher

A, D, E und L die oben angegebene Bedeutung haben,

durch Umsetzung mit (R)- oder (S)-Phenylethylamin in inerten Lösemitteln und anschließender Kristallisation der Phenethylammoniumsalze und anschließende Hydrolyse der Salze in die enantiomerenreinen Verbindungen der allgemeinen Formel (XIIIa,b)

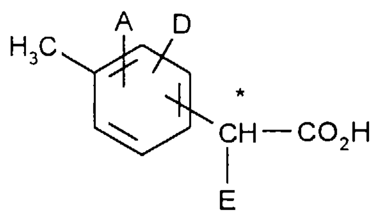

(XIIIa)

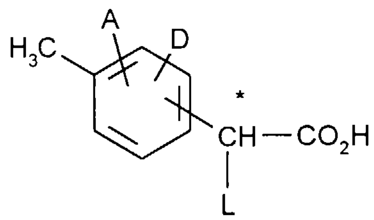

(XIIIb)

in welcher

A, D, E und L die oben angegebene Bedeutung haben,

überführt,
in einem weiteren Schritt mit Isobuten, in inerten Lösemitteln und in Anwesenheit von Säuren die enantiomerenreinen Ester der allgemeinen Formel (XIVa,b)

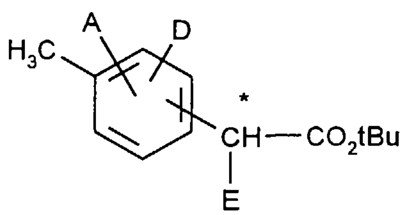

(XIVa)

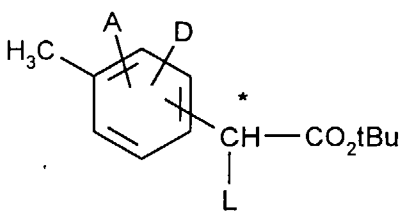

(XIVb)

in welcher

A, D, E und L die oben angegebene Bedeutung haben,

herstellt,
wie oben beschrieben durch eine Halogenierung in die enantiomerenreinen Verbindungen der allgemeinen Formel (XVa,b)

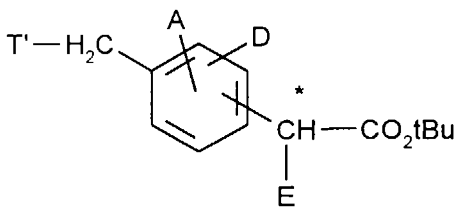

(XVa)

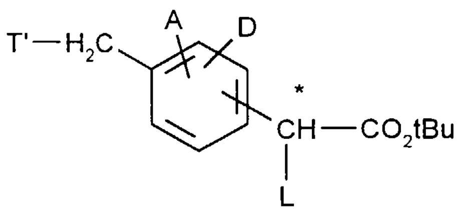

(XVb)

in welcher

A, D, T', E und L  die oben angegebene Bedeutung haben,

überführt, und durch Umsetzung mit den Verbindungen der allgemeinen Formel (V) in die enantiomerenreinen Ester der allgemeinen Formel (XVIa,b)

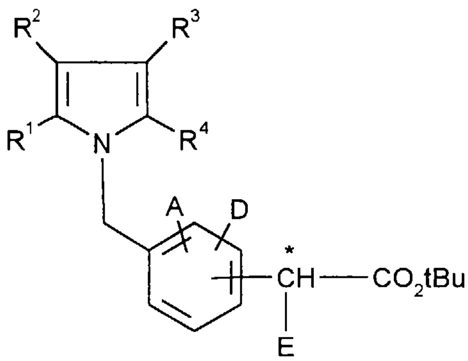

(XVIa)

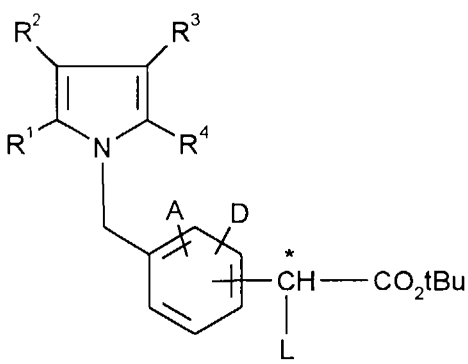

(XVIb)

in welcher

A, D, E, L, $R^1$, $R^2$, $R^3$ und $R^4$  die oben angegebene Bedeutung haben

überführt,
und in den letzten Schritten, wie vorne beschrieben, die entsprechenden enantiomerenreinen Säuren und aktivierten Derivate herstellt.

Als Lösemittel für die Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Dimethylformamid, Toluol und Tetrahydrofuran.

Als Basen für die erfindungsgemäßen Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate und -hydrogencarbonate wie Natriumcarbonat, Natriumhydrogencarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl($C_1$-$C_6$)amine) wie Triethylamin, oder Heterocyclen wie 1,4-Diazabicyclo[2,2,2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle wie Natrium oder deren Hydride wie Natriumhydrid einzusetzen. Bevorzugt sind Natriumhydrogencarbonat, Kaliumcarbonat und Kalium-tert.butylat, DBU oder DABCO.

Als Lösemittel eignen sich für die Hydrolyse Wasser oder die für eine Hydrolyse üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Hydrolyse kann gegebenenfalls auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Hydrolyse wird im allgemeinen in einem Temperaturbereich von 0°C bis +100°C, bevorzugt von +20°C bis +80°C durchgeführt.

Im allgemeinen wird die Hydrolyse bei Normaldruck durchgeführt. Es ist aber auch möglich, bei Unterdruck oder bei Überdruck zu arbeiten (z.B. von 0,5 bis 5 bar).

Bei der Durchführung der Hydrolyse wird die Base im allgemeinen in einer Menge von 1 bis 3 mol, bevorzugt von 1 bis 1,5 mol bezogen auf 1 mol des Esters, eingesetzt. Besonders bevorzugt verwendet man molare Mengen der Reaktanden.

Die Hydrolyse von tert.-Butylestern erfolgt im allgemeinen mit Säuren, wie beispielsweise Salzsäure oder Trifluoressigsäure, in Anwesenheit eines der oben angegebenen Lösemitteln und/oder Wasser oder deren Gemische, vorzugsweise mit Dioxan oder Tetrahydrofuran.

Das erfindungsgemäße allgemeine Verfahren wird im allgemeinen in einem Temperaturbereich von -30°C bis +200°C, bevorzugt von 80°C bis 150°C, durchgeführt.

Für die einzelnen Schritte zur Herstellung enantiomerenreiner Säuren eignen sich vorzugsweise folgende Bedingungen:

Die Herstellung der Verbindungen der allgemeinen Formeln (IXa) und (IXb) erfolgt vorzugsweise in Dimethylformamid und Kalium-tert.butanolat in einem Temperaturbereich von -10°C bis +10°C.

Die Halogenierung der Verbindungen der allgemeinen Formeln (Xa) und (Xb) wird in Chlorbenzol mit 1,3-Dibrom-5,5-dimethylhydantoin in Anwesenheit von Azobisisobutyronitril in einem Temperaturbereich von 0°C bis 110°C durchgeführt.

Die Umsetzung zu den Verbindungen der allgemeinen Formeln (XIa) und (XIb) erfolgt unter Schutzgasatmosphäre in Dimethylformamid und Kalium-tert.butanolat in einem Temperaturbereich von 0°C bis 30°C.

Die Hydrolyse der Verbindungen der allgemeinen Formeln (XIa) und (XIb) kann wie oben beschrieben durchgeführt werden, wobei das System HBr/Ameisensäure besonders bevorzugt ist. Die Verseifung wird in einem Temperaturbereich von 20°C bis 100°C durchgeführt.

Die Umsetzung der Verbindungen der allgemeinen Formel (XII) erfolgt mit Methylenchlorid unter Rückfluß.

Als aktivierende Reagenzien eignen sich vorzugsweise Trifluormethansulfonsäurechlorid, Mesylchlorid, Oxalylchlorid und Thionylchlorid. Besonders bevorzugt ist Thionylchlorid.

Die Umsetzung zu den Verbindungen der allgemeinen Formeln (XIVa) und (XIVb) erfolgt im ersten Schritt bevorzugt in Tetrahydrofuran und Triethylamin` im zweiten Schritt im System Wasser/Salzsäure. Die Reaktion wird in einem Temperaturbereich von 30°C bis 70°C durchgeführt.

Als Säuren für die Herstellung der erfindungsgemäßen Verbindungen der allgemeinen Formeln (XVa) und (XVb) wird besonders bevorzugt konzentrierte Schwefelsäure eingesetzt. Die Herstellung wird mit Methylenchlorid durchgeführt.

Im weiteren Aufarbeitungsgang wird als Base Kaliumcarbonat eingesetzt. Die Umsetzung erfolgt in einem Temperaturbereich von 0°C bis +20°C, besonders bevorzugt bei 10°C.

Die Halogenierung der Verbindungen der allgemeinen Formeln (XVa) und (XVb) erfolgt mit N-Bromsuccinimid in Methylenchlorid in Anwesenheit von Azobisisobutyronitril.

Im allgemeinen setzt man die Base in einer Menge von 0,05 mol bis 10 mol, bevorzugt von 1 mol bis 2 mol, jeweils bezogen auf 1 mol der Verbindungen der allgemeinen Formeln (IV), (VIIIa), (VIIIb), (XIa), (XIb), (XIVa) und (XIVb) ein.

Die erfindungsgemäßen Verfahren werden im allgemeinen bei Normaldruck durchgeführt. Es ist aber auch möglich, die Verfahren bei Überdruck oder bei Unterdruck durchzuführen (z.B. in einem Bereich von 0,5 bis 5 bar).

Die Verbindungen der allgemeinen Formel (III) sind an sich bekannt.

Die Verbindungen der allgemeinen Formeln (IV), (VIIIa) und (VIIIb) sind bekannt oder können in Analogie zu bekannten Methoden hergestellt werden.

Die Verbindungen der allgemeinen Formel (V) sind teilweise bekannt oder neu und können dann aber in Analogie zu publizierten Methoden hergestellt werden.

Die Verbindungen der allgemeinen Formel (VII) sind als Species neu und werden aus der entsprechenden Säure hergestellt.

Die enantiomerenreinen Verbindungen der allgemeinen Formeln (IXa) und (IXb) sind mit Ausnahme von X = CH-isopropyl neu und können wie oben beschrieben hergestellt werden.

Die Verbindungen der allgemeinen Formeln (Xa), (Xb), (XIa), (XIb) und (XII) sind neu und können wie oben beschrieben hergestellt werden.

Die Verbindungen der allgemeinen Formeln (XIIIa), (XIIIb), (XIVa) und (XIVb) sind teilweise bekannt oder nach üblichen Methoden herstellbar.

Die enantiomerenreinen Verbindungen der allgemeinen Formeln (XVa), (XVb), (XVIa) und (XVIb) sind neu und können wie oben beschrieben hergestellt werden.

Die Verbindungen der allgemeinen Formel (VI) sind neu und können wie oben beschrieben hergestellt werden.

Die erfindungsgemäßen Verbindungen der allgemeinen Formel (I) haben ein nicht vorhersehbares pharmakologisches Wirkspektrum.

Sie können als Wirkstoffe in Arzneimitteln zur Reduzierung von Veränderungen an Gefäßwänden Verwendung finden und zur Behandlung von koronaren Herzerkrankungen, Herzinsuffizienz, Störungen der Hirnleistung, ischämischen Gehirnerkrankungen, Apoplexie, Durchblutungsstörungen, Mikrozirkulationsstörungen und Thrombosen.

Weiterhin spielt bei der Okklusion von Gefäßen die Proliferation glatter Muskelzellen eine ausschlaggebende Rolle. Die erfindungsgemäßen Verbindungen sind geeignet, diese Proliferation zu inhibieren und damit atherosklerotische Prozesse zu verhindern.

Die erfindungsgemäßen Verbindungen zeichnen sich durch eine Senkung der ApoB-100-assoziierten Lipoproteine (VLDL und seiner Abbauprodukte, wie z.B. LDL), des ApoB-100, der Triglyceride und des Cholesterins aus. Damit besitzen sie wertvolle, im Vergleich zum Stand der Technik überlegene pharmakologische Eigenschaften.

Überraschenderweise besteht die Wirkung der erfindungsgemäßen Verbindungen zunächst in einer Verminderung oder vollständigen Inhibierung der Bildung und/oder der Freisetzung von ApoB-100-assoziierten Lipoproteinen aus Leberzellen, was eine Senkung des VLDL-Plasmaspiegels zur Folge hat. Diese VLDL-Senkung muß mit einer Senkung der Plasmaspiegel von ApoB-100, LDL, Triglyceriden und von Cholesterin einhergehen; es werden also gleichzeitig mehrere der obengenannten Risikofaktoren gesenkt, die an Gefäßwandveränderungen beteiligt sind.

Die erfindungsgemäßen Verbindungen können daher zur Präventation und Behandlung von Atherosklerose, der Fettsucht, Pankreatitis und der Obstipation eingesetzt werden.

**1. Hemmung der Freisetzung ApoB-100-assoziierter Lipoproteine**

Der Test zum Nachweis der Hemmung der Freisetzung ApoB-100-assoziierter Liproproteine aus Leberzellen erfolgte in vitro mit kultivierten Leberzellen, bevorzugt mit Zellen der humanen Linie HepG2. Diese Zellen werden unter Standardbedingungen in Medium für die Kultur eukariontischer Zellen gezüchtet, bevorzugt in RPMI 1640 mit 10% fötalem Kälberserum. HepG2-Zellen synthetisieren und sezernieren in den Kulturüberstand ApoB-100-assoziierte Lipoproteinpartikel, die im Prinzip ähnlich aufgebaut sind wie die VLDL- bzw. LDL-Partikel, die im Plasma zu finden sind.

Diese Partikel können mit einem Immunoassay für humanes LDL nachgewiesen werden. Dieser Immunoassay erfolgt mit Antikörpern, die im Kaninchen gegen humanes LDL unter Standardbedingungen induziert worden waren. Die anti-LDL-Antikörper (Kan-anti-LDL-Ak) wurden an einem Immunosorbens mit humanem LDL affinitätschromatographisch gereinigt. Diese gereinigten Kan-anti-LDL-Ak werden an die Oberfläche von Plastik adsorbiert. Zweckmäßigerweise erfolgt diese Adsorbtion an die Plastikoberfläche von Mikrotitierplatten mit 96 Vertiefungen, bevorzugt an MaxiSorp-Platten. Wenn im Überstand von Hep-G2-Zellen ApoB-100-assoziierte Partikel vorhanden sind, dann können diese an die insolubilisierten Kan-anti-LDL-Ak binden, und es entsteht ein Immunkomplex, der an die Plastikoberfläche gebunden ist. Nicht gebundene Proteine werden durch Waschen entfernt. Der sich an der Plastikoberfläche befindliche Immunkomplex wird mit monoklonalen Antikörpern nachgewiesen, die nach Standardbedingungen gegen humanes LDL induziert und gereinigt worden waren. Diese Antikörper wurden mit dem Enzym Peroxidase konjugiert. Peroxidase setzt das farblose Substrat TMB in Gegenwart von $H_2O_2$ in ein gefärbtes Produkt um. Nach Ansäuerung

des Reaktionsgemisches mit $H_2SO_4$ wird die spezifische Lichtadsorption bei 450 nm bestimmt, die ein Maß für die Menge von ApoB-100-assoziierten Partikeln ist, die von den HepG2-Zellen in den Kulturüberstand sezerniert worden waren.

Überraschenderweise hemmen die erfindungsgemäßen Verbindungen die Freisetzung der ApoB-100-assoziierten Partikel. Der $IC_{50}$-Wert gibt an, bei welcher Substanzkonzentration die Lichtadsorption im Vergleich zur Kontrolle (Lösemittelkontrolle ohne Substanz) um 50% inhibiert ist.

| Bsp.-Nr. | Apo B $IC_{50}$ [nM] |
|---|---|
| 2 | 1,1 |
| 6 | 0,8 |
| 10 | 1,0 |
| 16 | 8,9 |
| 26 | 1,8 |

**2. Bestimmung der VLDL-Sekretion in vivo am Hamster**

Der Effekt der Testsubstanzen auf die VLDL-Sekretion in vivo wird am Hamster untersucht. Hierzu werden Goldhamster nach Prämedikation mit Atropin (83 mg/kg s.c.) mit Ketavet (83 mg/kg s.c.) und Nembutal (50 mg/kg i.p.) narkotisiert. Wenn die Tiere reflexfrei geworden sind, wird die V. jugularis freipräpariert und kanüliert. Anschließend werden 0,25 ml/kg einer 20%igen Lösung von Triton WR-1339 in physiologischer Kochsalzlösung appliziert. Dieses Detergens hemmt die Lipoproteinlipase und führt so zu einem Anstieg des Triglyceridspiegels aufgrund eines ausbleibenden Katabolismus von sezernierten VLDL-Partikeln. Dieser Triglyceridanstieg kann als Maß für die VLDL-Sekretionsrate herangezogen werden. Den Tieren wird vor sowie ein und zwei Stunden nach Applikation des Detergens durch Punktion des retroorbitalen Venenplexus Blut entnommen. Das Blut wird zwei Stunden bei Raumtemperatur, anschließend über Nacht bei 4°C inkubiert, um die Gerinnung vollständig abzuschließen. Danach wird 5 Minuten bei 10.000 g zentrifugiert. Im so erhaltenen Serum wird die Triglyceridkonzentration mit Hilfe eines modifizierten kommerziell erhältlichen Enzymtests bestimmt (Merckotest® Triglyceride Nr. 14354). 100 µl Serum werden mit 100 µl Testreagenz in 96-Lochplatten versetzt und 10 Minuten bei Raumtemperatur inkubiert. Anschließend wird die optische Dichte bei einer Wellenlänge von 492 nM in einem automatischen Platten-Lesegerät bestimmt (SLT-Spectra). Serumproben mit einer zu hohen Triglyceridkonzentration werden mit physiologischer Kochsalzlösung verdünnt. Die in den Proben enthaltene Triglyceridkonzentration wird mit Hilfe einer parallel gemessenen Standardkurve bestimmt. Testsubstanzen werden in diesem Modell entweder unmittelbar vor Applikation des Detergens intravenös verabreicht oder vor Einleitung der Narkose oral oder subkutan.

**3. Hemmung der intestinalen Triglyceridabsorption in vivo (Ratten)**

Die Substanzen, die auf ihre triglyceridabsorptionshemmende Wirkung in vivo untersucht werden sollen, werden männlichen Wistar-Ratten mit einem Körpergewicht zwischen 170 und 230 g oral verabreicht. Zu diesem Zweck werden die Tiere 18 Stunden vor der Substanzapplikation in Gruppen zu 6 Tieren eingeteilt und anschließend wird ihnen das Futter entzogen. Trinkwasser steht den Tieren ad libitum zur Verfügung. Die Tiere der Kontrollgruppen erhalten eine wäßrige Traganth-Suspension bzw. eine Traganth-Suspension die Olivenöl enthält. Die Traganth-Olivenöl-Suspension wird mit dem Ultra-Turrax hergestellt. Die zu untersuchenden Substanzen werden in einer entsprechenden Traganth-Olivenöl-Suspension ebenfalls mit dem Ultra-Turrax, direkt vor der Substanzapplikation suspendiert.

Jeder Ratte wird vor der Schlundsondenapplikation zur Bestimmung des basalen Serumtriglyceridgehaltes Blut durch Punktion des retroorbitalen Venenplexus entnommen. Anschließend werden die Traganth-Suspension, die Traganth-Olivenöl-Suspensionen ohne Substanz (Kontrolltiere), bzw. die Substanzen, suspendiert in einer entsprechenden Traganth-Olivenöl-Suspension, den nüchternen Tieren mit einer Schlundsonde verabreicht. Die weiteren Blutentnahmen zur Bestimmung des postprandialen Serumtriglyceridanstiegs erfolgen in der Regel 1, 2 und 3 Stunden nach der Schlundsondenapplikation.

Die Blutproben werden zentrifugiert und nach Gewinnung des Serums die Triglyceride photometrisch mit einem EPOS-Analyzer 5060 (Eppendorf Gerätebau, Netheler & Hinz GmbH, Hamburg) bestimmt. Die Bestimmung der Triglyceride erfolgt vollenzymatisch mit einem handelsüblichen UV-Test.

Der postprandiale Setumtriglyceridanstieg wird durch Subtraktion des Triglyceridvorwertes jeden Tieres von seinen korrespondierenden postprandialen Triglyceridkonzentrationen (1, 2 und 3 Stunden nach Applikation) ermittelt.

Die Differenzen (in mmol/l) zu jedem Zeitpunkt (1, 2 und 3 Stunden) werden in den Gruppen gemittelt, und die Mittelwerte des Serumtriglyceridanstiegs (ΔTG) der substanzbehandelten Tiere mit den Tieren verglichen, die nur die Traganth-Öl-Suspension erhielten.

Ebenso wird der Serumtriglyceridverlauf der Kontrolltiere, die nur Traganth erhielten, berechnet. Der Substanzeffekt zu jedem Zeitpunkt (1, 2 oder 3 Stunden) wird wie folgt ermittelt und in Δ% von der ölbelasteten Kontrolle angegeben.

$$\Delta\%\ \text{Triglyceridanstieg} = \frac{\Delta TG_{\text{Substanz}} - \Delta TG_{\text{Traganthkontrolle}}}{\Delta TG_{\text{Ölbelastung}} - \Delta TG_{\text{Traganthkontrolle}}} \times 100$$

Effekt von 1, 3 oder 10 mg Prüfsubstanz / kg KG p.o. auf den Triglyceridanstieg (Δ%) 2 h nach einer Triglyceridbelastung im Serum nüchterner Ratten. Der Serumtriglyceridanstieg fettbelasteter Kontrolltiere bezogen auf den Serumtriglyceridspiegel von Traganth-Kontrolltieren entspricht 100%. n = 6 Tiere pro Gruppe.

| | Serumtriglyceridanstieg in % (2 h pp) |
|---|---|
| Triglyceridbelastung | 100 |
| Traganthkontrolle | 0 |

Die statistische Auswertung erfolgt mit Student's t-Test nach vorheriger Überprüfung der Varianzen auf Homogenität.

Substanzen, die zu einem Zeitpunkt den postprandialen Serumtriglyceridanstieg, verglichen mit dem der unbehandelten Kontrollgruppe, statistisch signifikant ($p < 0{,}05$) um mindestens 30 % vermindern, werden als pharmakologisch wirksam angesehen.

**4. Hemmung der VLDL-Sekretion in vivo (Ratte)**

Die Wirkung der Testsubstanzen auf die VLDL-Sekretion wird ebenfalls an der Ratte untersucht. Dazu wird Ratten 500 mg/kg Körpergewicht Triton WR-1339 (2,5 mg/kg), gelöst in physiologischer Kochsalzlösung, intravenös in die Schwanzvene appliziert. Triton WR-1339 inhibiert die Lipoproteinlipase und führt somit durch Hemmung des VLDL-Katabolismus zu einem Anstieg des Triglycerid- und Cholesterinspiegels. Diese Anstiege können als Maß für die VLDL-Sekretionsrate herangezogen werden.

Den Tieren wird vor sowie zwei Stunden nach Applikation des Detergens durch Punktion des retroorbitalen Venenplexus Blut entnommen. Das Blut wird zur Gerinnung 1 h bei Raumtemperatur inkubiert und das Serum durch Zentrifugation mit 10 000 g für 20 s gewonnen. Anschließend werden die Triglyceride mittels eines handelsüblichen gekoppelten Enzymtests (Sigma Diagnostics®, Nr. 339) bei einer Wellenlänge von 540 nm photometrisch bestimmt. Die Messung erfolgt mit Hilfe eines ebenfalls gekoppelten Enzymtests (Boehringer Mannheim®, Nr. 1442350) bei einer Wellenlänge von 546 nm. Proben mit Triglycerid- bzw. Cholesterinkonzentrationen, die den Meßbereich der Methoden überschreiten, werden mit physiologischer Kochsalzlösung verdünnt. Die Ermittlung der jeweiligen Serumkonzentrationen erfolgt anhand parallel gemessener Standardreihen. Testsubstanzen werden unmittelbar nach der Tritoninjektion oral, intravenös oder subcutan appliziert.

Die Erfindung betrifft außerdem die Kombination von Pyridazino-, Pyrimido-, Pyrazino- und Triazino-indole der allgemeinen Formel (I) mit einem Glucosidase -und/oder Amylasehemmer zur Behandlung von familiärer Hyperlipidamien, der Fettsucht (Adipositas) und des Diabetes mellitus. Glucosidase- und/oder Amylasehemmer im Rahmen der Erfindung sind beispielsweise Acarbose, Adiposine, Voglibose, Miglitol, Emiglitate, MDL-25637, Camiglibose (MDL-73945), Tendamistate, AI-3688, Trestatin, Pradimicin-Q und Salbostatin. Bevorzugt ist die Kombination von Acarbose, Miglitol, Emiglitate oder Voglibose mit einer der oben aufgeführten erfindungsgemäßen Verbindungen der allgemeinen Formel (I).

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

**Verwendete Abkürzungen:**

| | |
|---|---|
| Ac | = Acetyl |
| AIBN | = Azobisisobutyronitril |
| Bn | = Benzyl |
| Bz | = Benzoyl |
| cDec | = cyclo-Decyl |
| CDI | = N-(3-Dimethylaminopropyl)-N-ethylcarbodiimid-Hydrochlorid |
| cDodec | = cyclo-Dodecyl |
| cHept | = cyclo-Heptyl |
| cHex | = cyclo-Hexyl |
| cNon | = cyclo-Nonyl |
| cOct | = cyclo-Octyl |
| cPent | = cyclo-Pentyl |
| cPr | = cyclo-Propyl |
| 18-Crown-6 | = 1,4,7,10,13,16-Hexaoxacyclooctadecan |
| DCC | = Dicyclohexylcarbodiimid |
| DDQ | = 2,3-Dichlor-5,6-dicyano-1,4-benzochinon |
| dia | = Diastereomer |
| dia A | = Diastereomer mit dem größeren $R_f$-Wert |
| dia B | = Diastereomer mit dem kleineren $R_f$-Wert |
| DMAP | = 4-(N,N-Dimethylamino)pyridin |
| DME | = 1,2-Dimethoxyethan |
| DMF | = N,N-Dimethylformamid |
| DMSO | = Dimethylsulfoxid |
| ent | = Enantiomer |
| Et | = Ethyl |
| HOBT | = 1-Hydroxy-1H-benzotriazol |
| iBu | = isoButyl |
| iPent | = isoPentyl |
| iPr | = isoPropyl |
| Me | = Methyl |
| Ment | = Menthyl |
| Mes | = Mesyl |
| NBS | = N-Bromsuccinimid |
| nBu | = normalButyl |
| nHex | = normalHexyl |
| nPent | = normalPentyl |
| nPr | = normalPropyl |
| Ph | = Phenyl |
| PPA | = Polyphosphorsäure |

| | |
|---|---|
| pTol | = paraTolyl |
| pTos | = paraTosyl |
| rac | = Racemat |
| sBu | = sekundärButyl |
| tBu | = tertiärButyl |
| TFA | = Trifluoressigsäure |
| THF | = Tetrahydrofuran |
| TMS | = Tetramethylsilan |

**Die verwendeten Lösemittelgemische:**

| Solvens | Bezeichnung |
|---|---|
| Petrolether : Essigsäureethylester = 20:1 | A |
| Petrolether : Essigsäureethylester = 2:1 | B |
| Petrolether : Essigsäureethylester = 5:1 | C |
| Dichlormethan : Ethanol = 20:1 | D |
| Petrolether : Essigsäureethylester = 1:1 | E |
| Dichlormethan : Ethanol = 50:1 | F |
| Dichlormethan | G |
| Petrolether : Essigsäuremethylester = 9:1 | H |
| Dichlormethan : Methanol = 20:1 | I |
| Petrolether : Essigsäureethylester = 4:1 | J |
| Dichlormethan : Ethanol = 10:1 | K |
| Dichlormethan : Methanol = 100:3 | L |
| Toluol | M |
| Toluol : Essigester = 9:1 | N |
| Toluol : Essigsäureethylester = 2:1 | O |
| Petrolether : Essigester = 10:1 | P |
| Petrolether : Essigester = 20:1 | Q |
| Petrolether | R |
| Petrolether : Essigsäureethylester = 1:2 | S |
| Cyclohexan : Essigsäureethylester = 1:2 | T |
| Cyclohexan : Essigsäureethylester = 1:4 | U |

**Ausgangsverbindungen**

**Beispiel I**

4-Tolyl-essigsäure-methylester

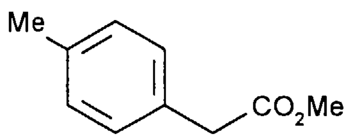

300 g (1,998 mol) 4-Tolyl-essigsäure werden in 2,5 l Methanol gelöst, mit 100 ml konz. Schwefelsäure verrührt und 2,5 h unter Rückfluß gekocht. Darauf rührt man nach und nach insgesamt 430 g (5,1 mol) Natriumhydrogencarbonat ein (Kohlendioxidentwicklung !), dampft das Methanol im Vakuum weitgehend ab, verteilt zwischen Wasser und Dichlormethan und extrahiert die wäßrige Phase mit Dichlormethan nach. Die vereinigten organischen Phasen werden mit Natriumsulfat getrocknet und im Vakuum vom Lösemittel befreit. Der Rückstand wird im Hochvakuum destilliert.

Ausbeute: 336 g
Siedetemperatur: 65°C (0,5 mbar)
$R_f$ = 0,81 (Toluol : Essigsäureethylester = 2:1)

**Beispiel II**

4-Tolyl-essigsäureethylester

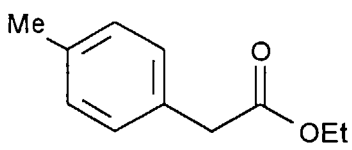

Ausgehend von 4-Tolyl-essigsäure wird der 4-Tolyl-essigsäureethylester analog der Vorschrift aus Beispiel I hergestellt.

$R_f$ = 0,43 (A)

**Beispiel III**

4-Methylphenylessigsäure-tert.butylester

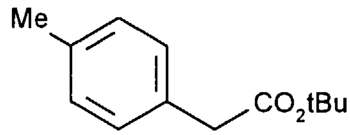

450 g (3 mol) 4-Methylphenylessigsäure, 1,13 l (12 mol) tert.Butanol und 90 g (0,74 mol) Dimethylaminopyridin werden in 2 l Dichlormethan gelöst. Nach Zugabe von 680 g (3,3 mol) Dicyclohexylcarbodiimid, gelöst in 400 ml Dichlormethan, wird 20 h bei 25°C gerührt. Der ausgefallene Harnstoff abgesaugt, mit 200 ml Dichlormethan gewaschen und die organische Phase je zweimal mit 500 ml 2 M Salzsäure und Wasser gewaschen. Die organische Phase wird mit Natriumsulfat getrocknet, eingeengt und destilliert.

Ausbeute: 408 g (66%)
Siedepunkt: 73-78°C (0,2 Torr)

**Beispiel IV**

2(R/S)-2-Cyclopentyl-2-(4-methylphenyl)essigsäure-tert.butylester

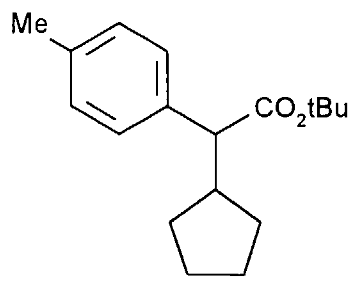

33,5 g (0,3 mol) Kalium-tert.butylat werden in 100 ml DMF unter Feuchtigkeitsausschluß bei 0°C vorgelegt, und 51,6 g (0,25 mol) der Verbindung aus Beispiel III in 250 ml DMF zugetropft. Es wird 30 min bei 0°C gerührt, 32,2 ml (0,3 mol) Cyclopentylbromid in 150 ml DMF bei 5-15°C zugetropft und 20 h bei 25°C gerührt. Nach Einengen wird der Rückstand zwischen Wasser/Diethylether verteilt, die Etherphase über Natriumsulfat getrocknet und eingeengt. Das Produkt kristallisiert aus.

Ausbeute: 67 g (97,5%)
Festpunkt: 51-53°C

Die racemischen Verbindungen der Tabelle I werden analog der Vorschrift von Beispiel IV hergestellt:

**Tabelle I:**

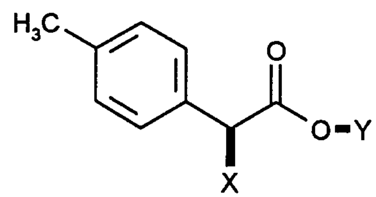

| Bsp.-Nr. | ▬X | ▬Y | a) Fp. (°C) b) $R_f$ (Solvens) | Spektren | Ausgangsmaterial aus Bsp.-Nr. |
|---|---|---|---|---|---|
| V | ▬$CH_3$ = Me | ▬$C(CH_3)_3$ = tBu | b) 0,71 (M) | | III |
| VI | ▬$C_2H_5$= Et | tBu | b) 0,67 (M) | | III |
| VII | ▬$CH_2CH_2CH_3$ = nPr | tBu | b) 0,69 (M) | | III |
| VIII | ▬$CH(CH_3)_2$ = iPr | Me | b) 0,86 (O) | | I |
| IX | ▬$CH(CH_3)_2$ = iPr | tBu | | | III |
| X | ▬$CH_2CH_2CH_2CH_3$ = nBu | tBu | b) 0,74 (M) | | III |
| XI | ▬$CH_2CH(CH_3)_2$ = iBu | tBu | b) 0,70 (M) | | III |
| XII | ▬$CH_2CH_2CH_2CH_2CH_3$ = nPent | tBu | | | III |
| XIII | ▬$CH_2CH_2$-$CH(CH_3)_2$ = iPent | tBu | b) 0,54 (P) | | III |

| Bsp.-Nr. | —X | —Y | a) Fp. (°C)<br>b) $R_f$ (Solvens) | Spektren | Ausgangsmaterial aus Bsp.-Nr. |
|---|---|---|---|---|---|
| XIV | $-CH(CH_2CH_3)_2$ | tBu | | MS: 276 ($M^+$, 4%) | III |
| XV | $-CH_2CH_2CH_2CH_2CH_2CH_3$ = nHex | tBu | b) 0,75 (M) | | III |
| XVI | $-CH_2CH(CH_2CH_3)_2$ | tBu | | MS: 290 ($M^+$, 1%) | III |
| XVII | 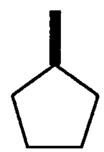 = cPent | Me | b) 0,59 (P) | | I |
| XVIII | 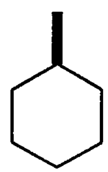 = cHex | Me | b) 0,62 (Q) | | I |
| XIX | cHex | tBu | b) 0,72 (M) | | III |

| Bsp.-Nr. | ▬X | ▬Y | a) Fp. (°C)<br>b) $R_f$ (Solvens) | Spektren | Ausgangsmaterial aus Bsp.-Nr. |
|---|---|---|---|---|---|
| XX | 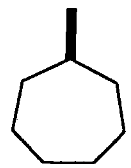 = cHept | Me | b) 0,57 (M) | | I |
| XXI | cHept | tBu | b) 0,67 (M) | | III |
| XXII | 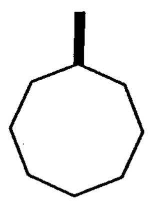 = cOct | tBu | b) 0,77 (M) | | III |
| XXIII | 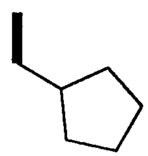 | tBu | b) 0,86 (Q) | | III |

| Bsp.-Nr. | —X | —Y | a) Fp. (°C)<br>b) $R_f$ (Solvens) | Spektren | Ausgangsmaterial aus Bsp.-Nr. |
|---|---|---|---|---|---|
| XXIV | 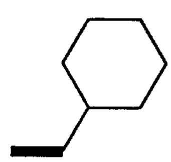 | tBu | | | III |

Die Verbindungen der Tabelle II werden analog der Vorschrift von Beispiel IV hergestellt; es werden lediglich 2,5 Äquivalente der Base und 2,5 Äquivalente des Halogenalkans (im Falle der cyclischen Alkylreste 1,2 Äquivalente des α,ω-Dihalogenalkans) eingesetzt.

**Tabelle II**

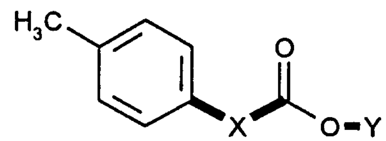

| Bsp.-Nr. | 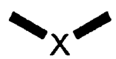 | 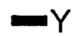 | a) Fp. (°C)<br>b) $R_f$ (Solvens) | Spektren | Ausgangsmaterial aus Bsp.-Nr. |
|---|---|---|---|---|---|
| XXV | 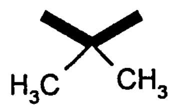 | tBu | b) 0,68 (F) | | III |
| XXVI | 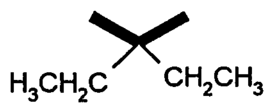 | tBu | b) 0,32 (R) | | III |
| XXVII | 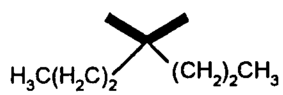 | tBu | b) 0,84 (B) | | III |

| Bsp.-Nr. | 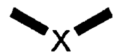 | 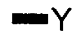 | a) Fp. (°C)<br>b) $R_f$ (Solvens) | Spektren | Ausgangsmaterial aus Bsp.-Nr. |
|---|---|---|---|---|---|
| XXVIII | 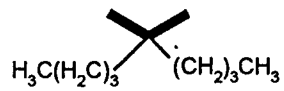 | tBu | b) 0,82 (C) | | III |
| XXIX | 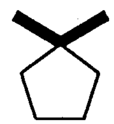 | tBu | b) 0,23 (R) | | III |
| XXX | 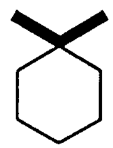 | tBu | b) 0,21 (R) | | III |
| XXXI | 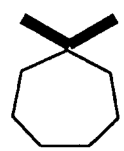 | tBu | b) 0,26 (R) | | III |

**Beispiel XXXII**

2(R/S)-2-(4-Brommethyl-phenyl)2-cyclopentyl-essisäure-tert.butylester

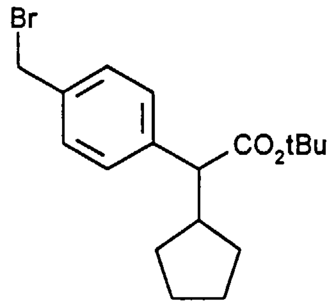

27,4 g (0,1 mol) der Verbindung aus Beispiel IV werden in 200 ml Tetrachlorkohlenstoff gelöst und zum Sieden erhitzt. Nach Zugabe von 0,82 g Azobisisobutyronitril werden 18,7 g (0,105 mol) N-Bromsuccinimid portionsweise zugegeben und anschließend 1 h refluxiert, auf 0°C abgekühlt und vom Succinimid abfiltriert. Nach Einengen des Filtrates fällt das Produkt aus. Es wird mit Petrolether (40/60) gewaschen und getrocknet.

Ausbeute: 20 g (57%)
Festpunkt: 73-76°C

Die racemischen Verbindungen der Tabelle III werden analog der Vorschrift von Beispiel-Nr. XXXII hergestellt:

**Tabelle III**:

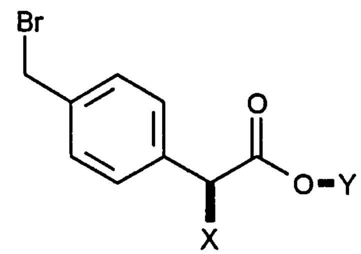

| Bsp.-Nr. | —X | —Y | a) Fp. (°C)<br>b) $R_f$ (Solvens) | Spektren | Ausgangsmaterial aus Bsp.-Nr. |
|---|---|---|---|---|---|
| XXXIII | —H | Me | | | I |
| XXXIV | —H | tBu | | | III |
| XXXV | Me | tBu | b) 0,78 (M) | | V |
| XXXVI | Et | tBu | b) 0,75 (M) | | VI |
| XXXVII | nPr | tBu | b) 0,80 (M) | | VII |
| XXXVIII | iPr | Me | b) 0,78 (G) | | VIII |
| XXXIX | iPr | tBu | | | IX |
| XL | nBu | tBu | b) 0,82 (M) | | X |
| XLI | iBu | tBu | b) 0,86 (G) | | XI |
| XLII | nPent | tBu | b) 0,73 (H) | | XII |

| Bsp.-Nr. | —X | —Y | a) Fp. (°C)<br>b) $R_f$ (Solvens) | Spektren | Ausgangsmaterial aus Bsp.-Nr. |
|---|---|---|---|---|---|
| XLIII | iPent | tBu | | MS: 372, 374 (([M+$NH_4$]$^+$; 79%, 77%) | XIII |
| XLIV | —$CH(CH_2CH_3)_2$ | tBu | | MS: 372, 372 ([M+$NH_4$]$^+$; 4%, 4%) | XIV |
| XLV | nHex | tBu | b) 0,85 (M) | | XV |
| XLVI | —$CH_2CH(CH_2CH_3)_2$ | tBu | | MS: 386, 388 ([M+$NH_4$]$^+$; 14%, 14%) | XVI |
| XLVII | cPent | Me | b) 0,63 (P) | | XVII |
| XLVIII | cHex | Me | b) 0,47 (Q) | | XVIII |
| IL | cHex | tBu | b) 0,58 (P) | | XIX |
| L | cHept | Me | b) 0,59 (M) | | XX |
| LI | cHept | tBu | b) 0,84 (G) | | XXI |
| LII | cOct | tBu | b) 0,49 (Q) | | XXII |

| Bsp.-Nr. | —X | —Y | a) Fp. (°C) b) $R_f$ (Solvens) | Spektren | Ausgangsmaterial aus Bsp.-Nr. |
|---|---|---|---|---|---|
| LIII | 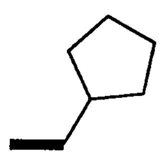 | tBu | b) 0,58 (A) | | XXIII |
| LIV | 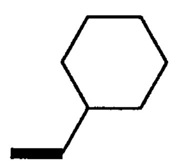 | tBu | | | XXIV |

Die Verbindungen der Tabelle IV werden analog der Vorschrift von Beispiel XXXI hergestellt:

**Tabelle IV**

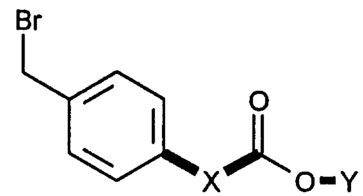

| Bsp.-Nr. | 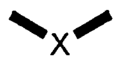 | 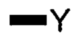 | a) Fp. (°C)<br>b) $R_f$ (Solvens) | Spektren | Ausgangsmaterial aus Bsp.-Nr. |
|---|---|---|---|---|---|
| LV | 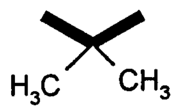 | tBu | b) 0,68 (F) | | XXV |
| LVI | 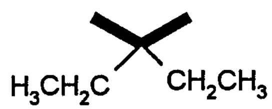 | tBu | b) 0,38 (Q) | | XXVI |
| LVII | 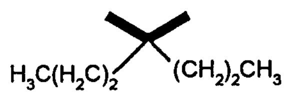 | tBu | b) 0,84 (B) | | XXVII |

| Bsp.-Nr. | 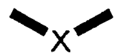 | 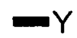 | a) Fp. (°C)<br>b) $R_f$ (Solvens) | Spektren | Ausgangsmaterial aus Bsp.-Nr. |
|---|---|---|---|---|---|
| LVIII | 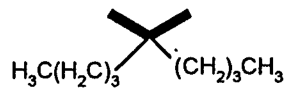 | tBu | b) 0,82 (C) | | XXVIII |
| LIX | 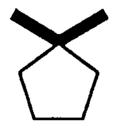 | tBu | | MS: 356, 358 ($[M+NH_4]^+$; 9%, 11%) | XXIX |
| LX | 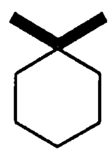 | tBu | | MS: 370, 372 ($[M+NH_4]^+$; 5%, 5%) | XXX |
| LXI | 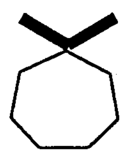 | tBu | b) 0,47 (Q) | | XXXI |

**Beispiel LXII**

1,3-Dimethyl-5-nitro-6-[2-(pyrrolidin-1-yl)-cyclohexen-1-yl]-pyrimidin-2,4(1H,3H)dion

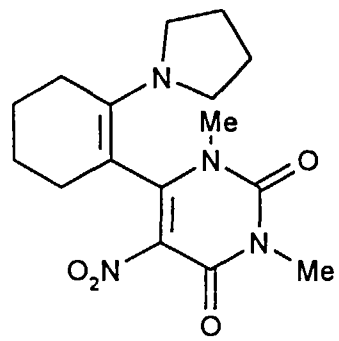

107 g (0,487 mol) 6-Chlor-1,3-dimethyl-5-nitro-pyrimidin-2,4(1H,3H)-dion [J. Clark und I.W. Southon, J. Chem. Soc. Perkin Trans. I, 1814 (1974)] werden in 700 ml wasserfreiem DMF gelöst und bei 20°C mit 81,8 g (0,974 mol) Natriumhydrogencarbonat und 73,66 g (0,487 mol) 1-(Cyclohexan-1-yl)-pyrrolidin verrührt. Nach 20 Stunden wird das Reaktionsgemisch in Ether und wäßrigen Puffer vom pH = 7 (Merck) eingerührt. Durch Dichlormethanzugabe werden die Phasen getrennt, die organische Phase mit Magnesiumsulfat getrocknet und eingedampft.

Ausbeute: 110 g (0,329 mol).
$R_f$=0,34 (F)
MS (DCI / $NH_3$): m/z = 335 (26%, $[M+H]^+$).

**Beispiel LXIII**

1,3-Dimethyl-1,5,6,7,8,9-hexahydro-pyrimidi[5,4-b]indol-2,4-dion

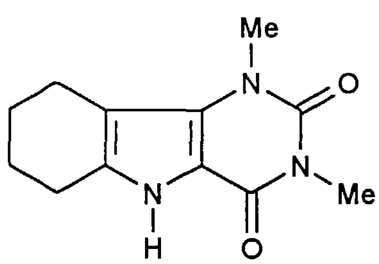

36,6 g (0,109 mmol) der Verbindung aus Beispiel LXII werden in 1,57 l Essigsäureethylester und Methanol gelöst und an 36 g Hydrierkatalysator (5% Palladium auf Tierkohle) bei 20°C und ca. 1 bar $H_2$-Druck reduziert. Nach einem anfänglichen Temperaturanstieg auf ca. 30°C wird die Reaktionsmischung 6 Stunden nach Reakionsbeginn über einen Seitzfilter abgesaugt und das Filtrat eingedampft. Das Rohprodukt wird nacheinander mit Methanol, wäßrigem Puffer vom pH = 2 (Merck) und Wasser verrührt, jeweils abgesaugt und schließlich im Vakuum über Phosphorpentoxid getrocknet (Ausbeute: 9,84 g (42,2 mmol)). Die methanolische Lösung wird eingedampft, das erhaltene Material zu den vereinigten wäßrigen Phasen gegeben und mit Diethylether extrahiert. Die organische Phase wird eingedampft und wiederum mit Methanol und Wasser verrührt. Nach Absaugen und Vakuumtrocknung über Phosphorpentoxid fallen weitere 0,22 g (0,9 mmol) Produkt an. Die Hydrierkatalysatorrückstände werden mit Dichlormethan und Methanol ausgekocht und abgesaugt. Das eingedampfte Filtrat wird wie oben behandelt und liefert nach Trocknung 0,81 g (3,5

mmol) der Titelverbindung.

$R_f$=0,29 (D)
MS (DCI / $NH_3$): m/z = 234 (100%, $[M+H]^+$).

**Beispiel LXIV**

1,3-Dimethyl-1,5-dihydro-pyrimido[5,4-b]indol-2,4-dion

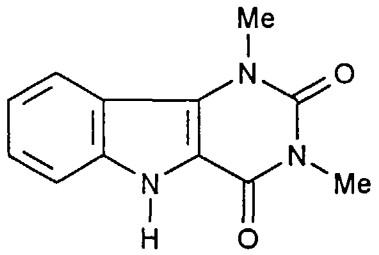

5,0 g (22,4 mmol) der Verbindung aus Beispiel LXIII werden insgesamt 42,5 Stunden mit 5 g Palladium (5%ig auf Tierkohle) und 7,5 ml Fumarsäurediethylester in 40 ml Diethylenglycol bei Rückflußtemperatur umgesetzt, wobei nach 10 und 20 Stunden die genannten Reagenz- und Lösemittelmengen noch einmal zugegeben werden. Die auf 20°C abgelcuhlte Reaktionsmischung wird mit je 500 ml Dichlormethan und Methanol versetzt und zum Rückfluß erhitzt. Diese heiße Mischung wird über einen Seitzfilter abgesaugt und mit 500 ml einer Lösemittelmischung aus Diethylether, Dichlormethan, Methanol und Essigsäureethylester (Mischungsverhältnis = 1:1:1:1) nachgewaschen. Das Filtrat wird eingedampft und mit Aceton ausgerührt. Der Niederschlag wird abgesaugt und im Hochvakuum vom Restlösemittel befreit.

Ausbeute: 1,9 g (8,3 mmol)
$R_f$ =0,38 (E)
MS (EI): m/z = 230 (100%, $[M+H]^+$).

**Beispiel LXV**

2,4-Dimethyl-5H-pyrimido[4,5-b]indol

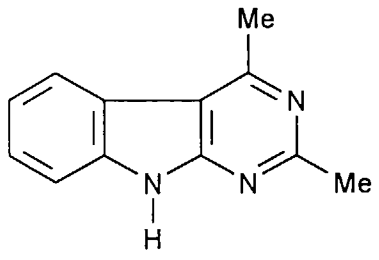

12,1 g (60,0 mmol) 2,4-Dimethyl-6,7,8,9-tetrahydro-5H-pyrimido[4,5-b]indol [T.D. Duffy und D.G. Wibberley, J. Chem. Soc., Perkin Trans. I,1921 (1974)] werden analog der Herstellungsvorschrift für die Verbindung aus Beispiel LXIV zu der bereits auf anderem Wege dargestellten [Y. Kondo, R. Watanabe, T. Sakamoto und H. Yamanaka, Chem. Pharm. Bull., 37, 2933 (1989)] Titelverbindung umgesetzt.

Ausbeute: 10,1 g (51,2 mmol)
$R_f$=0,20 (D)
MS (DCI / $NCH_3$) : m/z = 198 (100%, $[M+H]^+$).

**Beispiel LXVI**

3-Methoxycarbonyl-indol-2-carbonsäure

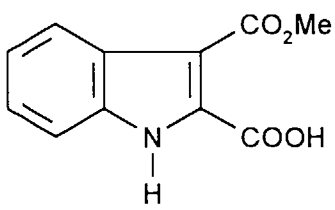

4,24 g (18,2 mmol) Indol-2,3-dicarbonsäure-dimethylester (Lancaster) werden in 120 ml Methanol gelöst und 6 Stunden mit 37 ml 2M wäßriger Natronlauge bei 20°C gerührt. Darauf gießt man auf Diethylether, Dichlormethan und Wasser, stellt durch Salzsäurezugabe auf pH = 3, extrahiert die wäßrige Phase mit dem Lösemittelgemisch nach, trocknet die vereinigten organischen Extrakte mit Magnesiumsulfat und dampft zur Trockne ein.

Ausbeute: 3,98 g. Die Substanz wird ohne weitere Reinigung weiter umgesetzt. $R_f$ = 0,18 (F)
$^{13}C$-NMR ($d_6$-DMSO, 75 MHz, $d_5$-DMSO): $\delta$ = 52,67 (Q); 106,81 (S); 113,17 (D); 122,05 (D); 122,76 (D); 125,18 (D); 125,52 (S); 132,48 (S); 135,18 (S); 160,56 (S); 167,91(S) ppm.
MS (EI): m/z = 219 (100%, $M^+$)

**Beispiel LXVII**

N,N'-Dimethyl-3-methoxycarbonyl-2-indolcarbohydrazid

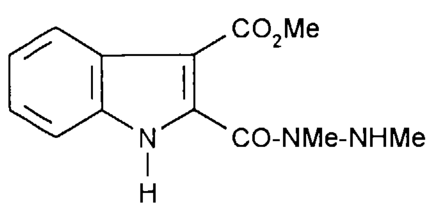

4,84 g (36,4 mmol) N,N'-Dimethylhydraziniumdichlorid werden in 60 ml Dichlormethan gelöst und bei 20°C nacheinander mit 5,1 ml Triethylamin, 3,99 g (18,2 mmol) der Verbindung aus Beispiel LXVI, 2,7 g (20 mmol) HOBT, 4,01 g (21 mmol) CDI und 7,6 ml (54,6 mmol) Triethylamin versetzt und 24 Stunden gerührt. Bei unvollständiger Umsetzung [DC-Kontrolle mit dem Laufmittelsystem Dichlormethan : Ethanol = 20:1] werden 1,35 g (10 mmol) HOBT, 2 g (10,5 mmol) CDI, 2,42 g (18,2 mmol) N,N'-Dimethylhydraziniumdichlorid und 3,8 g (27,3 mmol) Triethylamin zugegeben und weitere 24 Stunden gerührt. Der Ansatz wird auf Puffer vom pH = 2 (Merck) gegossen, die wäßrige Phase mit Dichlormethan nachextrahiert und die vereinigten organischen Phasen mit Magnesiumsulfat getrocknet und eingedampft. Nach chromatographischen Aufreinigung (Kieselgel 60, Merck, Dichlormethan : Ethanol, zuerst 50:1, dann 20:1, zuletzt 10:1) fallen 3,0 g (11,5 mmol) der Titelverbindung an.

$R_f$=0,34 (D)
MS (DCI/$NH_3$): m/z = 262 (100%, $[M+H]^+$).

**Beispiel LXVIII**

2,3-Dihydro-2,3-dimethyl-5H-pyridazino[4,5-b]indol-1,4-dion

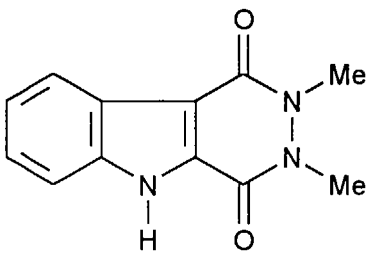

2,1 g (8,0 mmol) der Verbindung aus Beispiel LXVII werden 20 Stunden in 60 ml Tetralin bei 190°C gerührt. Nach Abkühlung auf 20°C fällt ein Niederschlag aus, der abgesaugt und mit Petrolether und Dichlormethan gewaschen wird. Die Entfernung von Lösemittelresten im Hochvakuum liefert eine Ausbeute von 1,3 g (5,7 mmol).

$R_f$ = 0,24 (D)
MS ($DCI/NH_3$): m/z = 230 (100%, $[M+H]^+$).

**Beispiel LXIX**

1,3-Dimethyl-1,9-dihydro-pyrimido[4,5-b]indol-2,4-dion

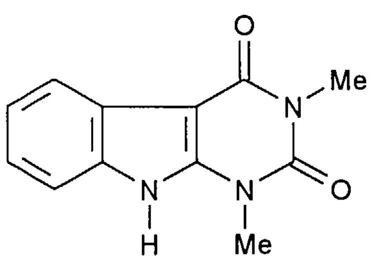

14,8 g (60,1 mmol) 1,3-Dimethyl-6-(N'-phenyl-hydrazino)-1H-pyrimidin-2,4-dion [S. Senda, K. Hirota und G.-N. Yang, Chem. Pharm. Bull. 20, 399 (1982)] werden in 180 ml Tetralin 30 Minuten unter Rückfluß gekocht [S. Senda, K. Hirota und M. Takahashi, J. Chem. Soc., Perkin Trans. I, 503 (1975)]. Nach Abkühlen auf 20°C fällt ein Niederschlag aus, der abgesaugt und mit Aceton gewaschen wird. Nach der Trocknung im Hochvakuum fallen 10,8 g (47,1 mmol) der Titelverbindung an.

$R_f$ = 0,34 (D)
MS (EI): m/z = 229 (100%, $M^+$)

**Beispiel LXX**

2-(R/S)-2-Cyclopentyl-2-(4-{1,3-dimethyl-1,5-dihydro-2,4-oxo-pyrimido[5,4-b]indol-5-yl-methyl}-phenyl)-essigsäure-(1,1-dimethyl-ethyl)-ester

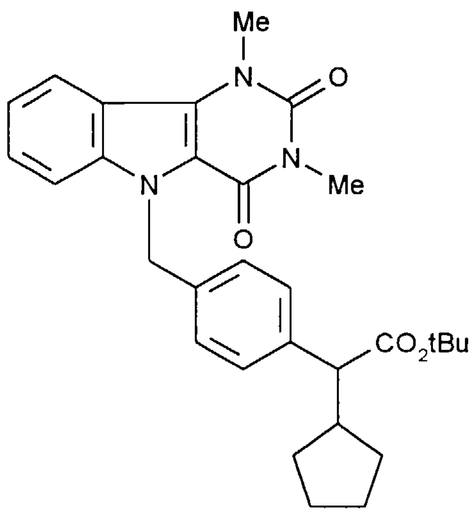

1,00 g (4,36 mmol) der Verbindung aus Beispiel LXIV wird bei ca. 20°C in 20 ml DMF mit 0,49 g (4,36 mmol) des Kaliumalkoholates des 1,1-Dimethyl-ethanols verrührt und nach 20 Minuten tropfenweise mit einer Lösung von 1,88 g (ca. 4,4 mmol / ca. 80%iges Material) 2-(R/S)2-(4-Brommethyl)-phenyl-2-cyclopentylessigsäure-(1,1-dimethyl-ethyl)-ester versetzt. Nach ca. 1 Stunde wird die Reaktionsmischung auf wäßrige Pufferlösung vom pH = 4 (Merck) gegossen und der anfallende Niederschlag abgesaugt (Sollte bei diesem Arbeitsschritt ein nicht absaugbares Material entstehen, so kann auch extraktiv aufgearbeitet werden). Das Rohprodukt wird an Kieselgel 60 (Merck / Petrolether : Essigsäureethylester = 1:1 bis 1:4) chromatographisch aufgereinigt.

Ausbeute: 0,485 g (0,97 mmol).
$R_f$ = 064 (E)

Die racemischen Verbindungen der Tabelle V werden analog der Vorschrift von Beispiel LXX hergestellt:

**Tabelle V:**

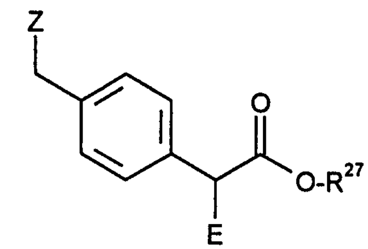

| Bsp.-Nr. | Z | (Absolute Konfiguration) E | $R^{27}$ | a) Fp. (°C) b) $R_f$ (Solvens) | Spektren | Ausgangsmaterial a) Literatur b) Vertreiberfirma c) Synthese analog / aus Bsp.-Nr. |
|---|---|---|---|---|---|---|
| LXXI | 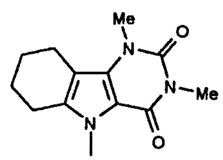 | (S) cPent | L-(-)-Ment | b) 0,25 (F) | | c) Bsp.-Nr. LXIII |
| LXXII | 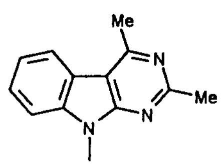 | (R+S) cPent | Me | b) 0,40 (D) | | c) Bsp.-Nr. LXV |

| Bsp.-Nr. | Z | (Absolute Konfiguration) E | $R^{27}$ | a) Fp. (°C) b) $R_f$ (Solvens) | Spektren | Ausgangsmaterial a) Literatur b) Vertreiberfirma c) Synthese analog / aus Bsp.-Nr. |
|---|---|---|---|---|---|---|
| LXXIII | 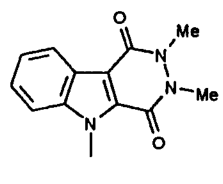 | (R+S) cPent | tBu | b) 0,35 (J) | | c) Bsp.-Nr. LXVIII |
| LXXIV | 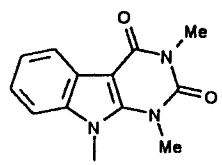 | (R+S) cPent | tBu | b) 0,27 (B) | MS (FAB): m/z = 524 (6%, $[M+Na]^+$), 501 (44%, $M^+$) | c) Bsp.-Nr. LXIX |
| LXXV | 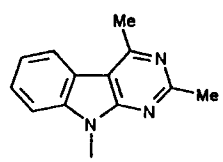 | (R+S) iPent | tBu | b) 0,45 (I) | MS (ESI): m/z = 472 (100%, $[M+H]^+$) | c) Bsp.-Nr. LXV |

| Bsp.-Nr. | Z | (Absolute Konfiguration) E | $R^{27}$ | a) Fp. (°C)<br>b) $R_f$ (Solvens) | Spektren | Ausgangsmaterial<br>a) Literatur<br>b) Vertreiberfirma<br>c) Synthese analog / aus Bsp.-Nr. |
|---|---|---|---|---|---|---|
| LXXVI | 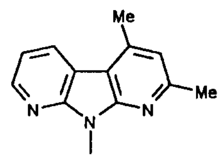 | (R+S) cPent | tBu | | | c) Bsp.-Nr. |
| LXXVII | 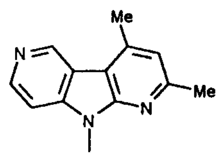 | (R+S) cPent | tBu | | | c) Bsp.-Nr. |
| LXXVIII | 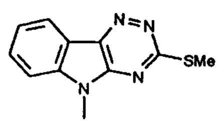 | (R+S) cPent | tBu | b) 0,27 (F) | | a) G. Doleschall und K. Lempert, Tetrahedron **30**, 3997 (1974). |
| LXXIX | 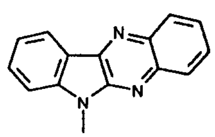 | (R+S) cPent | tBu | b) 0,57 (U) | | b) Parish |

**Beispiel LXXX**

2-(R/S)-2-Cyclopentyl-2-(4-{1,3-dimethyl-1,5-dihydro-2,4-oxo-pyrimido[5,4-b]indol-5-yl-methyl}-phenyl)-essigsäure

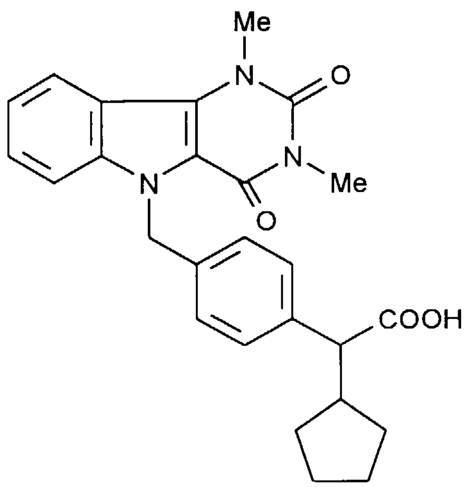

0,479 g (0,95 mmol) der Verbindung aus Beispiel LXX werden in 10 ml Dioxan gelöst, mit 0,84 ml konzentrierter Salzsäure versetzt und 6 Stunden in einem 70°C heißen Bad gerührt. Die Reaktionsmischung wird auf Wasser gegossen, mit 2 M wäßriger Natronlauge auf einen pH-Wert von1,6 gestellt, der so erhaltene Niederschlag abgesaugt und mit Wasser gewaschen. Nach Trocknung im Hochvakuum über Phosphorpentoxid erhält man 0,377 g (0,885 mmol) Produkt.

$R_f$ = 0,30 (D)

Die racemischen Verbindungen der Tabelle VI werden analog der Vorschrift von Beispiel LXXX hergestellt:

**Tabelle VI:**

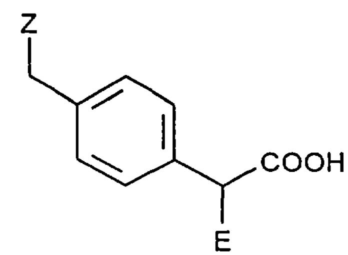

| Bsp.-Nr. | Z | (Absolute Konfiguration) E | a) Fp. (°C) b) $R_f$ (Solvens) | Spektren | Ausgangsmaterial a) Literatur b) Vertreiberfirma c) Synthese analog / aus Bsp.-Nr. |
|---|---|---|---|---|---|
| LXXXI | 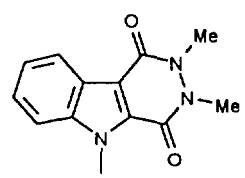 | (R+S) cPent | b) 0,45 (K) | | c) Bsp.-Nr. LXXIII |

| **Bsp.-Nr.** | **Z** | **(Absolute Konfiguration) E** | **a) Fp. (°C)<br>b) $R_f$ (Solvens)** | **Spektren** | **Ausgangsmaterial<br>a) Literatur<br>b) Vertreiberfirma<br>c) Synthese analog / aus Bsp.-Nr.** |
|---|---|---|---|---|---|
| LXXXII | 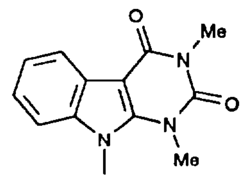 | (R+S) cPent | b) 0,23 (F) | | c) Bsp.-Nr. LXXIV |
| LXXXIII | 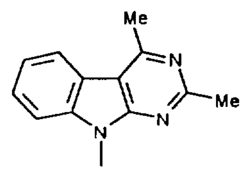 | (R+S) iPent | a) 198°C | MS (ESI): m/z = 416 (100%, $[M+H]^+$) | c) Bsp.-Nr. LXXV |
| LXXXIV | 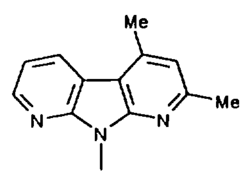 | (R+S) cPent | a) > 220°C | MS (ESI/pos.): m/z = 436 ($[M+H]^+$, 100%) | c) Bsp.-Nr. LXXVI |

| Bsp.-Nr. | Z | (Absolute Konfiguration) E | a) Fp. (°C) b) $R_f$ (Solvens) | Spektren | Ausgangsmaterial a) Literatur b) Vertreiberfirma c) Synthese analog / aus Bsp.-Nr. |
|---|---|---|---|---|---|
| LXXXV | 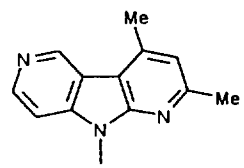 | (R+S) cPent | | | c) Bsp.-Nr. LXXVII |
| LXXXVI | 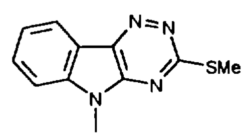 | (R+S) cPent | b) 0,27 (D) | | c) Bsp.-Nr. LXXVIII |
| LXXXVII | 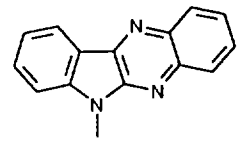 | (R+S) cPent | a) >220°C | MS (ESI/pos.): m/z = 436 ($[M+H]^+$, 100%) | c) Bsp.-Nr. LXXIX |

**Beispiel LXXXVIII**

2-(R/S)-2-Cyclopentyl-2-[4-{(2,4-dimethyl-5H-pyrimido[4,5-b]indol-5-yl)-methyl}-phenyl]-dihydrochlorid

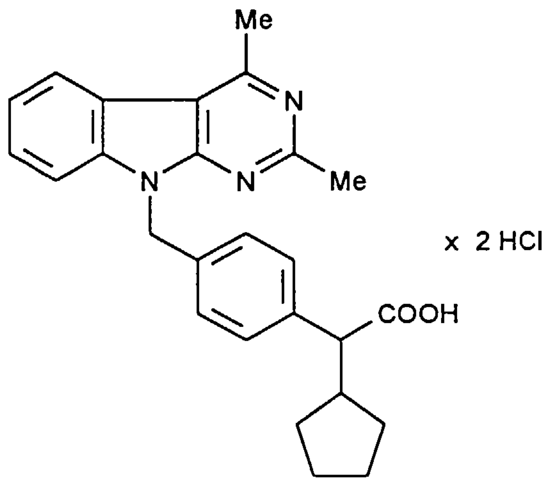

4,30 g (10,06 mmol) der Verbindung aus Beispiel LXXII werden 4 Stunden mit 0,264 g (1 mmol) 18-Krone-6 in 30,2 ml 1 M wäßriger Kalilauge und 40 ml Methanol unter Rückfluß gekocht. Nach Abkühlung der Reaktionsmischung werden zur Aufarbeitung 100 ml Wasser zugegeben und mit Diethylether extrahiert. Die wäßrige Phase wird mit 1 M Salzsäure auf einen pH-Wert von 2 gestellt, der anfallende Niederschlag abgesaugt und mit Wasser gewaschen. Die Hochvakuumtrocknung über Phosphorpentoxid und Kaliumhydroxid liefert 4,6 g (9,46 mmol) Produkt.

$R_f$= 0,22 (D)

**Beispiel LXXXIX**

2-(S)-2-Cyclopentyl-2-(4{1,3-dimethyl-1,5,6,7,8,9-hexahydro-2,4-oxopyrimido[5,4-b]indol-5-yl-methyl}-phenyl})-essigsäure

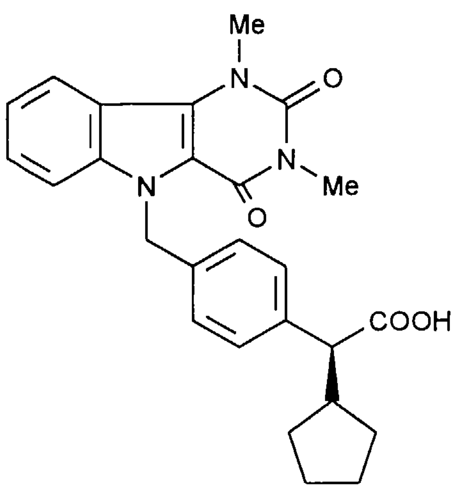

0,98 g (1,7 mmol) der Verbindung aus Beispiel LXXI werden 1,5 Stunden in 10 ml ca. 40%iger wäßriger Bromwasserstoffsäure und 20 ml Ameisensäure unter Rückfluß gekocht. Die tief rote Reaktionsmischung wird auf Diethylether und Wasser gegossen, die wäßrige Phase mit 2 M Natronlauge auf pH = 3 gestellt und mehrfach mit Diethylether nachextrahiert. Die vereinigten etherischen Extrakte werden mit Magnesiumsulfat getrocknet und eingedampft. Nach chromatographischer Aufreinigung an Kieselgel 60 (Merck / Petrolether : Essigsäureethylester = 1:1) fallen 0,176g (0,39 mmol) Produkt an.

$R_f$ = 0,18 (S)

**Herstellungsbeispiele**

**Beispiel-Nr. 1,2 und 3**

2-(R)- und 2-(S)-2-Cyclopentyl-2-(4-{1,3-dimethyl-1,5-dihydro-2,4-oxo-pyrimido-[5,4-b]indol-5-yl-methyl}-phenyl)-essigsäure-N-(2-hydroxy-1-(R)-1-phenyl-ethyl)amid

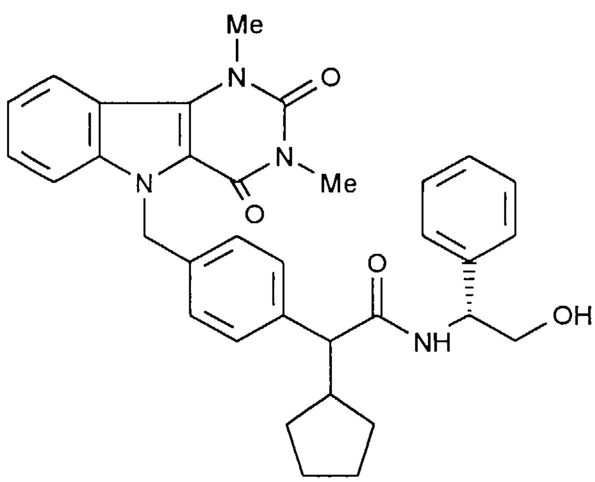

377 mg (0,846 mol) der Verbindung aus Beispiel LXXX werden bei 20°C mit 116 mg (0,846 mmol) 2-(R)-2-Amino-2-phenyl-ethanol, 126 ml (0,93 mmol) HOBT, 186,5 mg (0,97 mmol) CDI und 0,234 ml (1,7 mmol) Triethylamin in 20 ml Dichlormethan versetzt und 20 Stunden gerührt. Die Reaktionsmischung wird nacheinander mit wäßriger Ammoniumchloridlösung, wäßriger Natriumhydrogencarbonatlösung und wäßrigem Puffer vom pH 0,4 (Merck) extrahiert, mit Magnesiumsulfat getrocknet und im Vakuum eingedampft.

Ausbeute: 450 mg (0,80 mmol)

Beispiel-Nr. 1: Diasteromer A + B

$R_f$= 0,36 (D)
MS (FAB): m/z = 587 (37%, $[M+Na]^+$), 565 (100%, $[M+H]^+$).

Das Diastereomerengemisch wird chromatographisch an Kromasil 100 C 18 (55% einer 0,2%igen wäßrigen Trifluoressigsäurelösung + 45% Acetonitril) getrennt.

Beispiel-Nr. 2: Diastereomer A [2(S)-Diastereomer]:

$^1$H-NMR ($d_6$-DMSO, 200 MHz, TMS) charakt. Signale: $\delta$ = 6,95 - 7,12 (M, 6H); 7,15 - 7,26 (M, 3H) ppm.

Beispiel-Nr.3: Diastereomer B [2(R)-Diastereomer]:

$^1$H-NMR ($d_6$-DMSO, 200 MHz, TMS) charakt.Signale: $\delta$ = 7,08 (M, 2H); 7,16 - 7,33 (M, 8H) ppm.

Die absoluten Konfigurationen der enantiomerenreinen Carbonsäuren 2-(S)-und 2-(R)-2-{4-[(Chinolin-2-yl)methoxy-phenyl}-2-cyclopentyl-essigsäure [EP 509 359] sind bekannt, so daß die absoluten Konfigurationen der daraus analog der Vorschrift zu den Beispielen 1 und 2 hergestellten Amiden Bsp.-Nr. C1 und Bsp.-Nr. C2 abgeleitet werden können. Die $^1$H-NMR-Spektren der beiden diastereomeren Produkte (200 MHz, $d_6$-DMSO, TMS für Beispiel-Nr. C1 und 250 MHz, $d_6$-DMSO, TMS für Beispiel-Nr. C2 / Abbildung 1) weisen im Aromatenbereich signifikanate Unterschiede auf: die H-Signale des Phenylrestes von Beispiel-Nr. C1 bei ca. 7.1 ppm (3H) und 7.3 ppm (2H), die H-Signale von Beispiel-Nr. C2 bei ca. 7,3 ppm (5H). Dieser Befund ist auf die Verbindungen der Beispiele 2 und 3 sowie auf andere Derivate diesen Typs übertragen und die angegebenen absoluten und relativen Konfigurationen so ermittelt worden.

Die Verbindungen der Tabelle 1 werden analog der Vorschrift von Beispiel 1,2 und 3 hergestellt:

**Tabelle 1:**

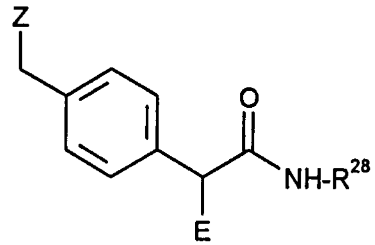

| Bsp.-Nr. | Z | (Absolute Konfiguration) E | $R^{28}$ | a) Fp. (°C)<br>b) $R_f$ (Solvens) | Spektren | Ausgangsmaterial<br>a) Literatur<br>b) Vertreiberfirma<br>c) Synthese analog / aus Bsp.-Nr. |
|---|---|---|---|---|---|---|
| 4 | 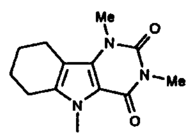 | (S) cPent | (R) 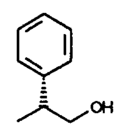 | b) 0,29 (D) | | c) Bsp.-Nr. LXXXIX |
| 5 | 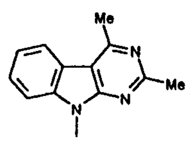 | (R+S) cPent | (R) 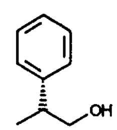 | b) 0,20 / 0,32 (D) | | c) Bsp.-Nr. LXXXVIII |

| Bsp.-Nr. | Z | (Absolute Konfiguration) E | $R^{28}$ | a) Fp. (°C) b) $R_f$ (Solvens) | Spektren | Ausgangsmaterial a) Literatur b) Vertreiberfirma c) Synthese analog / aus Bsp.-Nr. |
|---|---|---|---|---|---|---|
| 6 | 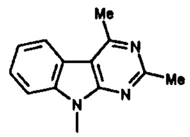 | (S) cPent | (R) 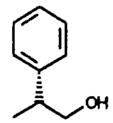 | b) 0,32 (D) | | c) Bsp.-Nr. LXXXVIII |
| 7 | 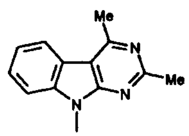 | (R) cPent | (R) 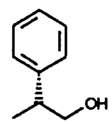 | b) 0,20 (D) | | c) Bsp.-Nr. LXXXVIII |
| 8 | 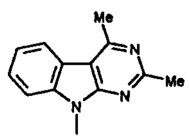 | (R+S) cPent | 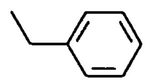 | b) 0,36 (D) | MS (ESI): m/z = 503 (100%, $[M+H]^+$) | c) Bsp.-Nr. LXXXVIII |
| 9 | 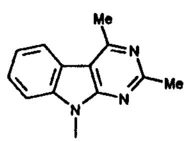 | (R+S) cPent | 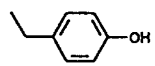 | b) 0,23 (D) | MS (ESI): m/z = 519 (100%, $[M+H]^+$) | c) Bsp.-Nr. LXXXVIII |

| Bsp.-Nr. | Z | (Absolute Konfiguration) E | $R^{28}$ | a) Fp. (°C) b) $R_f$ (Solvens) | Spektren | Ausgangsmaterial a) Literatur b) Vertreiberfirma c) Synthese analog / aus Bsp.-Nr. |
|---|---|---|---|---|---|---|
| 10 | 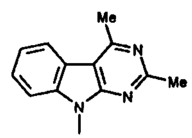 | (R+S) cPent | 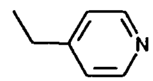 | b) 0,08 (D) | | c) Bsp.-Nr. LXXXVIII |
| 11 | 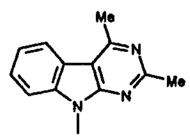 | (R+S) cPent | 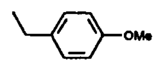 | b) 0,39 (D) | MS (ESI): m/z = 533 (100%, $[M+H]^+$) | c) Bsp.-Nr. LXXXVIII |
| 12 | 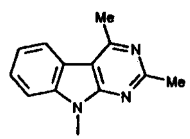 | (R+S) cPent | 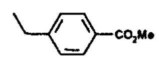 | b) 0,36 (D) | MS (ESI): m/z = 561 (100%, $[M+H]^+$) | c) Bsp.-Nr. LXXXVIII |
| 13 | 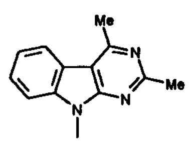 | (R+S) iPent | 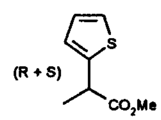 | a) 80°C b) 0,52 (I) | MS (ESI): m/z = 569 (100%, $[M+H]^+$) | c) Bsp.-Nr. LXXXIII |

| Bsp.-Nr. | Z | (Absolute Konfiguration) E | $R^{28}$ | a) Fp. (°C) b) $R_f$ (Solvens) | Spektren | Ausgangsmaterial a) Literatur b) Vertreiberfirma c) Synthese analog / aus Bsp.-Nr. |
|---|---|---|---|---|---|---|
| 14 | 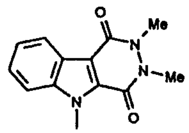 | (R+S) cPent | (R) 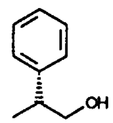 | b) 0,29 (D) | MS (FAB): m/z = 587 (11%, $[M+Na]^+$), 565 (43%, $[M+H]^+$) | c) Bsp.-Nr. LXXXI |
| 15 | 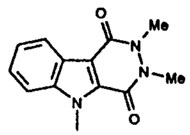 | (S) cPent | (R) 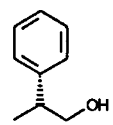 | b) 0,29 (D) | | c) Bsp.-Nr. LXXXI |
| 16 | 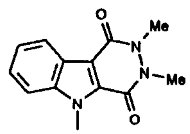 | (R) cPent | (R) 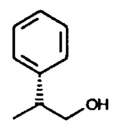 | b) 0,29 (D) | | c) Bsp.-Nr. LXXXI |
| 17 | 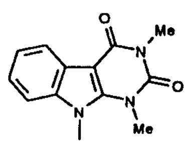 | (R+S) cPent | (R) 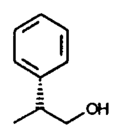 | b) 0,15 (F) | | c) Bsp.-Nr. LXXXII |

| Bsp.-Nr. | Z | (Absolute Konfiguration) E | $R^{28}$ | a) Fp. (°C) b) $R_f$ (Solvens) | Spektren | Ausgangsmaterial a) Literatur b) Vertreiberfirma c) Synthese analog / aus Bsp.-Nr. |
|---|---|---|---|---|---|---|
| 18 | 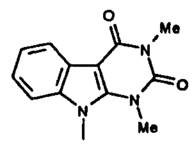 | (S) cPent | (R) 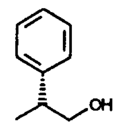 | b) 0,15 (F) | | c) Bsp.-Nr. LXXXII |
| 19 | 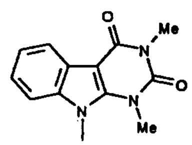 | (R) cPent | (R) 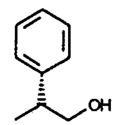 | b) 0,15 (F) | | c) Bsp.-Nr. LXXXII |
| 20 | 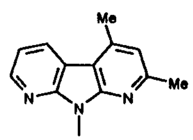 | (R+S) cPent | (R) 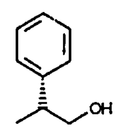 | | | c) Bsp.-Nr. LXXXIV |
| 21 | 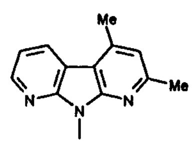 | (S) cPent | (R) 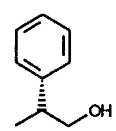 | | | c) Bsp.-Nr. LXXXIV |

| Bsp.-Nr. | Z | (Absolute Konfiguration) E | $R^{28}$ | a) Fp. (°C) b) $R_f$ (Solvens) | Spektren | Ausgangsmaterial a) Literatur b) Vertreiberfirma c) Synthese analog / aus Bsp.-Nr. |
|---|---|---|---|---|---|---|
| 22 | 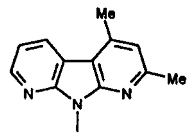 | (R) cPent | (R) 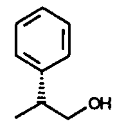 | | | c) Bsp.-Nr. LXXXIV |
| 23 | 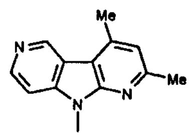 | (R+S) cPent | (R) 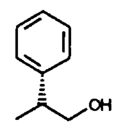 | | | c) Bsp.-Nr. LXXXV |
| 24 | 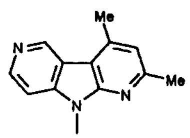 | (S) cPent | (R) 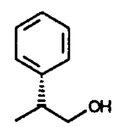 | | | c) Bsp.-Nr. LXXXV |
| 25 | 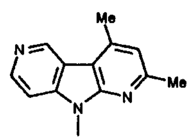 | (R) cPent | (R) 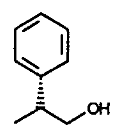 | | | c) Bsp.-Nr. LXXXV |

| Bsp.-Nr. | Z | (Absolute Konfiguration) E | $R^{28}$ | a) Fp. (°C)<br>b) $R_f$ (Solvens) | Spektren | Ausgangsmaterial<br>a) Literatur<br>b) Vertreiberfirma<br>c) Synthese analog /<br>aus Bsp.-Nr. |
|---|---|---|---|---|---|---|
| 26 | 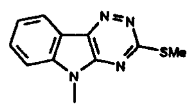 | (R+S) cPent | (R) 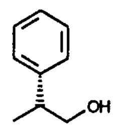 | b) 0,37 (D) | MS (FAB):<br>m/z = 552<br>(100%,<br>$[M+H]^+$) | c) Bsp.-Nr. LXXXVI |
| 27 | 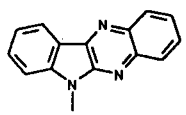 | (R+S) cPent | (R) 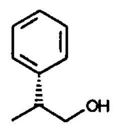 | a) 214°C<br>b) 0,50 (T) | | c) Bsp.-Nr. LXXXVII |

**Beispiel 28**

2(R/S)-2-Cyclopentyl-2-[4-{(2,4-dimethyl-5H-pyrimido[4,5-b]indol-5-yl)methyl}-phenyl-essigsäure-N-(4-carboxy-benzyl)-amid-dihydrochlorid

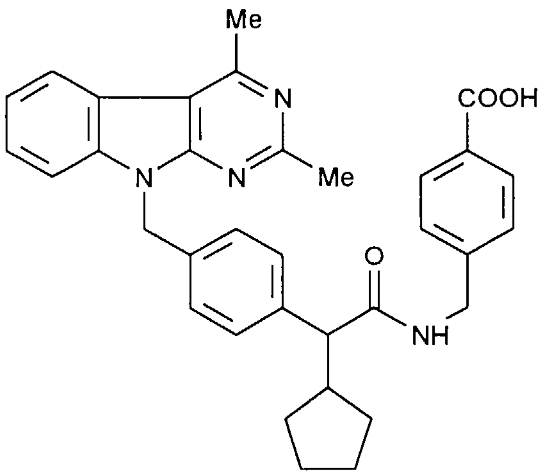

x 2 HCl

0,35 g (0,62 mmol) der Verbindung aus Beispiel 12 werden in 1 ml Methanol gelöst, mit 0,6 ml 2 M wäßriger Natronlauge versetzt und 2 Stunden unter Rückfluß gekocht. Die abgekühlte Reaktionsmischung wird mit 100 ml Wasser verdünnt und mit 2 ml Diethylether extrahiert. Darauf wird die wäßrige Phase mit 1 M wäßriger Salzsäure auf einen pH-Wert von 2 gestellt und der anfallende Niederschlag abgesaugt und mit Wasser gewaschen. Nach Hochvakuumtrocknung über Kaliumhydroxid fallen 0,32 g (0,52 mmol) Produkt an.

$R_f$=0,18 (D)
MS (CI): m/z = 547 (100%, $[M+H]^+$ / ohne HCl).

**Beispiel 29, Beispiel 30 und Beispiel 31**

9-(4-(1-(3-Methyl)butyl-1-1(R/S)-(2-hydroxy-1-thien-2-yl)ethylaminocarbonyl-4-methyl)-pentyl)benzyl-2,4-dimethyl-pyrimido[4,5-b]indol und die racemischen Diasteromere von 9-(4-(1-(2-Hydroxy-1-thien-2-yl)-ethylaminocarbonyl-4-methyl)-pentyl)benzyl-2,4-dimethyl-pyrimido[4,5-b]indol

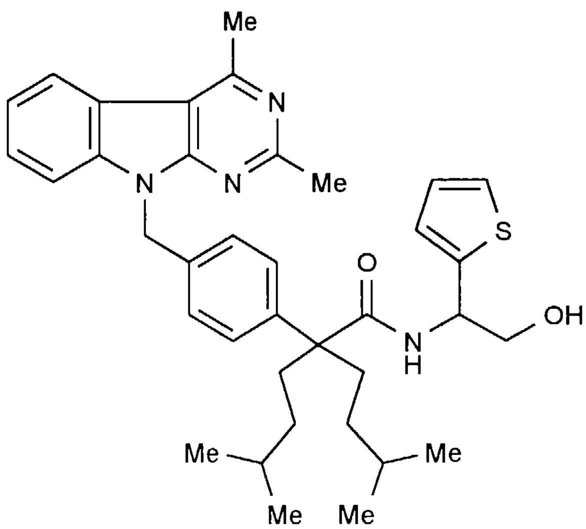

(29)

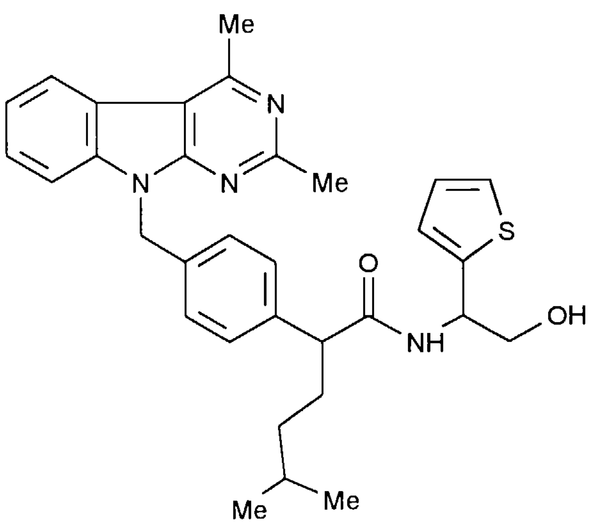

(30) und (31)

1,47 g (2,58 mmol) der Verbindung aus Beispiel 13 werden in 25 ml THF gelöst und bei 0°C mit 5,7 ml Lithiumaluminiumhydridlösung in THF (1M, 5,69 mmol) tropfenweise versetzt. Nach etwa 15 min rühren bei dieser Temperatur wird mit Wasser versetzt, mit Essigester extrahiert, die organische Phase mit Kalium-Natrium-Tartrat-Lösung gewaschen und über Natriumsulfat getrocknet. Chromatographie an Kieselgel mit einem Cyclohexan-Essigester 4:1 Gemisch lieferte drei Fraktionen:

Beispiel 29: 300 mg [19%]
Beispiel 30:146 mg [10%]
Beispiel 31: 57 mg [ 4%]

physikalische Daten:

**Beispiel 29:**

Fp.: 168°C
$R_f$ = 0,53 (U)
MS (ESI/POS): 611 ($[M+H]^+$, 100%)
$^1$H-NMR ($CDCl_3$, TMS): $\delta$ = 0,74 - 1,06 (m, 16H); 1,46 (sept., J = 6 Hz, 2H); 1,91 (t, breit, J = 7,5 Hz, 4H); 2,84 (s, 3H); 3,02 (s, 3H); 3,80 (d, J = 5 Hz, 2H); 5,32 (dt, $J_1$ = 7,5 Hz, $J_2$ = 5 Hz, 1H); 5,60 - 5,72 (m, 3H); 6,74 - 6,86 (m, 2H); 7,09 (d, breit, J = 5 Hz, 1H); 7,20 - 7,48 (m, 7H); 8,10 (d, J = 7,5 Hz, 1H) ppm.

**Beispiel 30:**

Fp.: 201°C
$R_f$ = 0,44 (U)
MS (CI, $NH_3$): 541 ($[M+H]^+$, 100%)
$^1$H-NMR ($CDCl_3$, TMS) $\delta$ = 0,81 (d, J = 7 Hz, 3H); 0,84 (d, J = 7 Hz, 3H); 0,94 - 1,28 (m, 2H); 1,50 (sept., J = 6 Hz, 1H); 1,58 - 1,82 (m, 1H); 2,00 - 2,21 (m, 1H); 2,81 (s, 3H); 2,98 (s, 3H); 3,27 (t, J = 7,5 Hz, 1H); 3,86 (d, J = 5 Hz, 2H); 5,29 (dt, $J_1$ = 7,5 Hz, $J_2$ = 5 Hz, 1H); 5,62 (s, 2H); 6,06 (d, J = 7,5 Hz, 1H); 6,65 - 6,84 (m, 2H); 7,05 (d, breit, J = 5 Hz, 1H); 7,16 - 7,50 (m, 7H); 8,08 (d, J = 9 Hz, 1H) ppm.

**Beispiel 31**:

Fp.: 194°C
$R_f$ = 0,36 (U)
MS (ESI/POS): 541 ([M+H]+, 100%)
$^1$H-NMR ($CDCl_3$, TMS) $\delta$ = 0,81 (d, J = 7 Hz, 3H); 0,83 (d, J = 7 Hz, 3H); 0,94 - 1,22 (m, 2H); 1,40 - 1,58 (m, 1H); 1,60 - 1,82 ((m, 1H); 1,98 - 2,19 (m, 1H); 2,82 (s, 3H); 2,97 (s, 3H); 3,25 (t, J= 7,5 Hz, 1H); 3,72 - 3,86 (m, 2H); 5,31 (dt, $J_1$ = 7,5 Hz, $J_2$ = 5 Hz, 1H); 5,62 (s, 2H); 6,08 (d, J = 7,5 H, 1H); 6,86 - 6,96 (m, 2H); 7,12 - 7,50 (m, 8H); 8,08 (d, J = 9 Hz, 1H) ppm.

**Patentansprüche**

1. Pyridazino-, Pyrimido-, Pyrazino- und Triazino-indole der allgemeinen Formel (I)

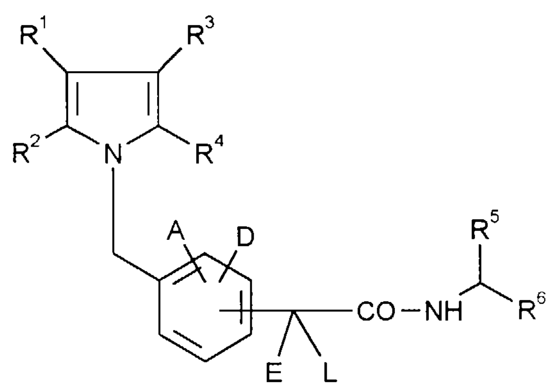

(I)

in welcher

$R^1$ und $R^2$ unter Einbezug der sie verbindenden Doppelbindung gemeinsam einen Phenylring oder einen 5- bis 8-gliedrigen Cycloalken- oder Oxocycloalken-Ring bilden,
der gegebenenfalls bis zu 3-fach gleich oder verschieden durch Halogen, Trifluormethyl, Carboxy, Hydroxy, durch geradkettiges oder verzweigtes Alkoxy oder Alkoxycarbonyl mit jeweils bis zu 6 Kohlenstoffatomen oder durch geradkettiges oder verzweigtes

Alkyl mit bis zu 6 Kohlenstoffatomen substituiert ist, das seinerseits durch Hydroxy oder durch geradkettiges oder verzweigtes Alkoxy mit bis zu 4 Kohlenstoffatomen substituiert sein kann,

$R^3$ und $R^4$ unter Einbezug der Doppelbindung gemeinsam einen Rest der Formel

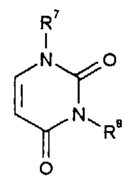 , 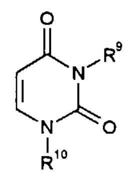 , 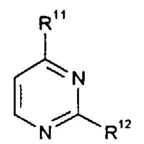 , 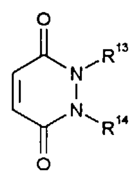 ,

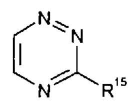 oder 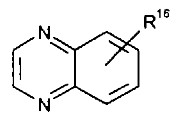

bilden,
worin

$R^7$, $R^8$, $R^9$, $R^{10}$, $R^{11}$, $R^{12}$, $R^{13}$, $R^{14}$, $R^{15}$ und $R^{16}$ gleich oder verschieden sind und Wasserstoff, Carboxyl geradkettiges oder verzweigtes Alkoxy, Alkylthio, Acyl oder Alkoxycarbonyl mit jeweils bis zu 6 Kohlenstoffatomen oder geradkettiges oder verzweigtes Alkyl mit bis zu 6 Kohlenstoffatomen bedeuten, das gegebenenfalls durch Hydroxy substituiert ist,

oder

$R^1$ und $R^2$ unter Einbezug der Doppelbindung einen Pyridylring bilden, und

$R^3$ und $R^4$ ebenfalls unter Einbezug der Doppelbindung gemeinsam einen Pyridylring bilden, wobei beide Pyridylringe gegebenenfalls bis zu 3-fach gleich oder verschieden durch Halogen, Trifluormethyl, Carboxy, Hydroxy, durch geradkettiges oder verzweigtes Alkoxy oder Alkoxycarbonyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy oder durch geradkettiges oder verzweigtes Alkoxy mit bis zu 4 Kohlenstoffatomen substituiert ist,

A und D gleich oder verschieden sind und
für Wasserstoff, Halogen, Trifluormethyl, Hydroxy oder geradkettiges oder verzweigtes Alkyl oder Alkoxy mit jeweils bis zu 5 Kohlenstoffatomen stehen,

E und L gleich oder verschieden sind und
für Wasserstoff, Cycloalkyl mit 3 bis 8 Kohlenstoffatomen oder geradkettiges oder verzweigtes Alkyl mit bis zu 10 Kohlenstoffatomen stehen, das gegebenenfalls durch Cycloalkyl mit 3 bis 6 Kohlenstoffatomen substituiert ist, oder
für Phenyl stehen, das gegebenenfalls durch Halogen oder Trifluormethyl substituiert ist, oder

E und L gemeinsam mit dem Kohlenstoffatom einen 4-8 gliedrigen Cycloalkylring bilden,

$R^5$ für Phenyl oder für einen 5- bis 7-gliedrigen gesättigten oder ungesättigten Heterocyclus mit bis zu 3 Heteroatomen aus der Reihe S, N und/oder O steht,
wobei die Cyclen gegebenenfalls bis zu 3-fach gleich oder verschieden durch Nitro, Carboxy, Halogen, Cyano oder durch geradkettiges oder verzweigtes Alkenyl oder Alkoxycarbonyl mit jeweils bis zu 6 Kohlenstoffatomen oder durch geradkettiges oder verzweigtes Alkyl mit bis zu 6 Kohlenstoffatomen substituiert sind, das gegebenenfalls durch Hydroxy, Carboxy oder durch geradkettiges oder verzweigtes Alkoxy oder Alkoxycarbonyl mit jeweils bis zu 6 Kohlenstoffatomen substituiert ist,
und/oder die Cyclen gegebenenfalls durch eine Gruppe der Formel $-OR^{17}$ oder $-NR^{18}R^{19}$ substituiert sind,
worin

$R^{17}$ Wasserstoff oder geradkettiges oder verzweigtes Alkyl oder Alkenyl mit jeweils bis zu 6 Kohlenstoffatomen bedeutet,

$R^{18}$ bzw. $R^{19}$ gleich oder verschieden sind und Phenyl, Wasserstoff oder geradkettiges oder verzweigtes Alkyl mit bis zu 6 Kohlenstoffatomen bedeuten
oder geradkettiges oder verzweigtes Acyl mit bis zu 8 Kohlenstoffatomen bedeuten, das gegebenenfalls durch eine Gruppe der Formel $-NR^{20}R^{21}$ substituiert ist,
worin

$R^{20}$ und $R^{21}$ gleich oder verschieden sind und Wasserstoff oder geradkettiges oder verzweigtes Acyl mit bis zu 8 Kohlenstoffatomen bedeuten,

$R^{6}$ für Wasserstoff, Carboxy oder für geradkettiges oder verzweigtes Alkoxycarbonyl mit bis zu 5 Kohlenstoffatomen steht,
oder für geradkettiges oder verzweigtes Alkyl mit bis zu 6 Kohlenstoffatomen steht, das gegebenenfalls durch Hydroxy oder durch eine Gruppe der Formel $-O-CO-R^{22}$ substituiert ist,
worin

$R^{22}$ Phenyl bedeutet, das gegebenenfalls bis zu 3-fach gleich oder verschieden durch Halogen, Hydroxy oder durch geradkettiges oder verzweigtes Alkyl mit bis zu 5 Kohlenstoffatomen substituiert ist,
oder geradkettiges oder verzweigtes Alkyl oder Alkenyl mit jeweils bis zu 22 Kohlenstoffatomen bedeutet, die gegebenenfalls durch eine Gruppe der Formel $-OR^{23}$ substituiert sind,
worin

$R^{23}$ Wasserstoff, Benzyl, Triphenylmethyl oder geradkettiges oder verzweigtes Acyl mit bis zu 6 Kohlenstoffatomen bedeutet,

gegebenenfalls in einer isomeren Form und deren Salze.

**2.** Pyridazino-, Pyrimido-, Pyrazino- und Triazino-indole der Formel nach Anspruch 1, in welcher

$R^1$ und $R^2$ unter Einbezug der sie verbindenden Doppelbindung gemeinsam einen Phenylring oder einen Cyclopenten-, Cyclohexen-, Cyclohepten-, Cycloocten-, Oxocyclopenten-, Oxocyclohexen-, Oxocyclohepten- oder Oxocycloocten-Rest bilden,
der gegebenenfalls bis zu 2-fach gleich oder verschieden durch Fluor, Chlor, Brom, Trifluormethyl, Carboxy, Hydroxy, durch geradkettiges oder verzweigtes Alkoxy oder Alkoxycarbonyl mit jeweils bis zu 4 Kohlenstoffatomen oder durch geradkettiges oder verzweigtes Alkyl mit bis zu 4 Kohlenstoffatomen substituiert ist, das seinerseits durch Hydroxy oder durch geradkettiges oder verzweigtes Alkoxy mit bis zu 3 Kohlenstoffatomen substituiert sein kann,

$R^3$ und $R^4$ unter Einbezug der Doppelbindung gemeinsam einen Rest der Formel

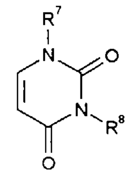 , 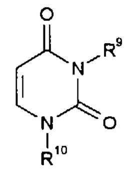 , 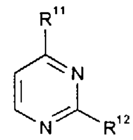 , 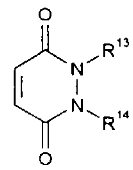 ,

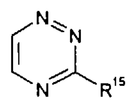 oder 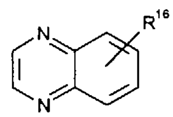

bilden,
worin

$R^7$, $R^8$, $R^9$, $R^{10}$, $R^{11}$, $R^{12}$, $R^{13}$, $R^{14}$, $R^{15}$ und $R^{16}$ gleich oder verschieden sind und Wasserstoff, geradkettiges oder verzweigtes Alkoxy, Alkylthio, Acyl oder Alkoxycarbonyl mit jeweils bis zu 4 Kohlenstoffatomen oder geradkettiges oder verzweigtes Alkyl mit bis zu 4 Kohlenstoffatomen bedeuten, das gegebenenfalls durch Hydroxy substituiert ist,

oder

$R^1$ und $R^2$ unter Einbezug der Doppelbindung einen Pyridylring bilden, und

$R^3$ und $R^4$ ebenfalls unter Einbezug der Doppelbindung gemeinsam einen Pyridylring bilden, wobei beide Pyridylringe gegebenenfalls bis zu 2-fach gleich oder verschieden durch Fluor, Chlor, Brom, Trifluormethyl, Carboxy, Hydroxy, durch geradkettiges oder verzweigtes Alkoxy oder Alkoxycarbonyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy oder durch geradkettiges oder verzweigtes Alkoxy mit bis zu 3 Kohlenstoffatomen substituiert ist,

A und D gleich oder verschieden sind und
für Wasserstoff, Fluor, Chlor, Brom, Trifluormethyl, Hydroxy oder geradkettiges oder verzweigtes Alkyl oder Alkoxy mit jeweils bis zu 4 Kohlenstoffatomen stehen,

E und L gleich oder verschieden sind und
für Wasserstoff, Cyclopropyl, Cyclobutyl, Cyclopentyl, Cyclohexyl, Cycloheptyl, Cyclooctyl oder für geradkettiges oder verzweigtes Alkyl mit bis zu 8 Kohlenstoffatomen stehen, das gegebenenfalls durch Cyclopropyl, Cyclopentyl oder Cyclohexyl substituiert ist, oder
für Phenyl stehen, das gegebenenfalls durch Fluor, Chlor oder Brom substituiert ist, oder

E und L gemeinsam mit dem Kohlenstoffatom einen 4-7 gliedrigen Cycloalkylring bilden,

$R^5$ für Phenyl, Pyridyl, Furyl, Thienyl oder Imidazolyl steht, die gegebenenfalls bis zu 2-fach gleich oder verschieden durch Nitro, Carboxy, Fluor, Chlor, Brom, Cyano, durch geradkettiges oder verzweigtes Alkenyl oder Alkoxycarbonyl mit jeweils bis zu 4 Kohlenstoffatomen oder durch geradkettiges oder verzweigtes Alkyl mit bis zu 5 Kohlenstoffatomen substituiert sind, das gegebenenfalls durch Hydroxy, Carboxy oder durch geradkettiges oder verzweigtes Alkoxy oder Alkoxycarbonyl mit jeweils bis zu 5 Kohlenstoffatomen substituiert ist,
und/oder die Cyclen gegebenenfalls durch eine Gruppe der Formel $-OR^{17}$ oder $-NR^{18}R^{19}$ substituiert sind,
worin

$R^{17}$ Wasserstoff oder geradkettiges oder verzweigtes Alkyl oder Alkenyl mit jeweils bis zu 4 Kohlenstoff-

atomen bedeutet,

$R^{18}$ und $R^{19}$ gleich oder verschieden sind und Phenyl, Wasserstoff oder geradkettiges oder verzweigtes Alkyl mit bis zu 5 Kohlenstoffatomen bedeuten,
oder geradkettiges oder verzweigtes Acyl mit bis zu 6 Kohlenstoffatomen bedeuten, das gegebenenfalls durch eine Gruppe der Formel $-NR^{20}R^{21}$ subsituiert ist,
worin

$R^{20}$ und $R^{21}$ gleich oder verschieden sind und Wasserstoff oder geradkettiges oder verzweigtes Acyl mit bis zu 6 Kohlenstoffatomen bedeuten,

$R^6$ für Wasserstoff, Carboxy oder für geradkettiges oder verzweigtes Alkoxycarbonyl mit bis zu 4 Kohlenstoffatomen steht, oder für geradkettiges oder verzweigtes Alkyl mit bis zu 5 Kohlenstoffatomen steht, das gegebenenfalls durch Hydroxyl oder durch eine Gruppe der Formel $-O-CO-R^{22}$ substituiert ist,
worin

$R^{22}$ Phenyl bedeutet, das gegebenenfalls bis zu 3-fach gleich oder verschieden durch Fluor, Chlor, Brom, Hydroxy oder durch geradkettiges oder verzweigtes Alkyl bis zu 4 Kohlenstoffatomen substituiert ist,
oder geradkettiges oder verzweigtes Alkyl oder Alkenyl mit jeweils bis zu 20 Kohlenstoffatomen bedeutet, die gegebenenfalls durch eine Gruppe der Formel $-OR^{23}$ substituiert sind,
worin

$R^{23}$ Wasserstoff, Benzyl, Triphenylmethyl oder geradkettiges oder verzweigtes Acyl mit bis zu 5 Kohlenstoffatomen bedeutet,

gegebenenfalls in einer isomeren Form und deren Salze.

**3.** Pyridazino-, Pyrimido-, Pyrazino- und Triazino-indole der Formel nach Anspruch 1,
in welcher

$R^1$ und $R^2$ unter Einbezug der sie verbindenden Doppelbindung gemeinsam einen Phenylring oder einen Cyclopenten-, Cyclohexen-, Cyclohepten-, Cycloocten-, Oxocyclopenten-, Oxocyclohexen-, Oxocyclohepten- oder Oxocycloocten-Rest bilden,
die gegebenenfalls bis zu 2-fach gleich oder verschieden durch Fluor, Chlor, Brom, Trifluormethyl, Carboxy, Hydroxy, durch geradkettiges oder verzweigtes Alkoxy oder Alkoxycarbonyl mit jeweils bis zu 3 Kohlenstoffatomen oder durch geradkettiges oder verzweigtes Alkyl mit bis zu 3 Kohlenstoffatomen substituiert sind, das seinerseits durch Hydroxy, Methoxy oder Ethoxy substituiert sein kann,

$R^3$ und $R^4$ unter Einbezug der Doppelbindung gemeinsam einen Rest der Formel

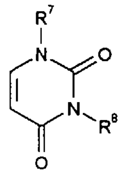 , 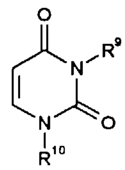 , 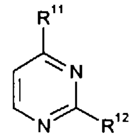 , 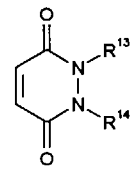

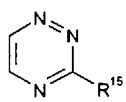 oder 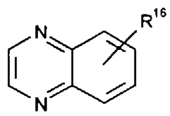

bilden,
worin

$R^7$, $R^8$, $R^9$, $R^{10}$, $R^{11}$, $R^{12}$, $R^{13}$, $R^{14}$, $R^{15}$ und $R^{16}$ gleich oder verschieden sind und Wasserstoff, geradkettiges oder verzweigtes Alkoxy oder Alkylthio mit jeweils bis zu 3 Kohlenstoffatomen oder geradkettiges oder verzweigtes Alkyl mit bis zu 3 Kohlenstoffatomen bedeuten, das gegebenenfalls durch Hydroxy substituiert ist,

oder

$R^1$ und $R^2$ unter Einbezug der Doppelbindung einen Pyridylring bilden, und

$R^3$ und $R^4$ ebenfalls unter Einbezug der Doppelbindung gemeinsam einen Pyridylring bilden, wobei beide Pyridylringe gegebenenfalls bis zu 2-fach gleich oder verschieden durch Fluor, Chlor, Brom, Trifluormethyl, Carboxy, Hydroxy, durch geradkettiges oder verzweigtes Alkoxy oder Alkoxycarbonyl mit jeweils bis zu 3 Kohlenstoffatomen oder durch geradkettiges oder verzweigtes Alkyl mit bis zu 3 Kohlenstoffatomen substituiert sind, das seinerseits durch Hydroxy, Methoxy oder Ethoxy substituiert ist,

A und D gleich oder verschieden sind und
für Wasserstoff, Fluor, Chlor, Brom oder Trifluormethyl stehen,

E und L gleich oder verschieden sind und
für Wasserstoff, Cyclopropyl, Cyclobutyl, Cyclopentyl, Cyclohexyl, Cycloheptyl, Cyclooctyl oder für geradkettiges oder verzweigtes Alkyl mit bis zu 6 Kohlenstoffatomen stehen, das gegebenenfalls durch Cyclopentyl oder Cyclohexyl substituiert ist, oder für Phenyl stehen, das gegebenenfalls durch Fluor, Chlor oder Brom substituiert ist, oder

E und L gemeinsam mit dem Kohlenstoffatom einen 5-7 gliedrigen Cycloalkylring bilden,

$R^5$ für Phenyl, Pyridyl oder Thienyl steht, die gegebenenfalls bis zu 2-fach gleich oder verschieden durch Nitro, Carboxy, Fluor, Chlor, Brom, Cyano, durch geradkettiges oder verzweigtes Alkenyl oder Alkoxycarbonyl mit jeweils bis zu 3 Kohlenstoffatomen oder durch geradkettiges oder verzweigtes Alkyl mit bis zu 4 Kohlenstoffatomen substituiert sind, das gegebenenfalls durch Hydroxy, Carboxy oder durch geradkettiges oder verzweigtes Alkoxy oder Alkoxycarbonyl mit jeweils bis zu 4 Kohlenstoffatomen substituiert ist,
und/oder die Cyclen gegebenenfalls durch eine Gruppe der Formel $-OR^{17}$ oder $-HR^{18}R^{19}$ substituiert sind,
worin

$R^{17}$ Wasserstoff oder geradkettiges oder verzweigtes Alkyl oder Alkenyl mit jeweils bis zu 3 Kohlenstoffatomen bedeutet,

$R^{18}$ und $R^{19}$ gleich oder verschieden sind und Phenyl, Wasserstoff oder geradkettiges oder verzweigtes Alkyl mit bis zu 4 Kolllenstoffatomen bedeuten,
oder geradkettiges oder verzweigtes Acyl mit bis zu 5 Kohlenstoffatomen bedeuten, das gegebenenfalls durch eine Gruppe der Formel $-NR^{20}R^{21}$ substituiert ist,
worin

$R^{19}$ und $R^{20}$ gleich oder verschieden sind und Wasserstoff oder geradkettiges oder verzweigtes Acyl mit bis zu 5 Kohlenstoffatomen bedeuten,

$R^6$ für Wasserstoff, Carboxy oder für geradkettiges oder verzweigtes Alkoxycarbonyl mit bis zu 3 Kohlenstoffatomen steht,
oder für geradkettiges oder verzweigtes Alkyl mit bis zu 4 Kohlenstoffatomen steht, das gegebenenfalls durch Hydroxyl oder durch eine Gruppe der Formel $-O-CO-R^{22}$ substituiert ist,
worin

$R^{22}$ Phenyl bedeutet, das gegebenenfalls bis zu 3-fach gleich oder verschieden durch geradkettiges oder verzweigtes Alkyl mit bis zu 3 Kohlenstoffatomen substituiert ist,
oder geradkettiges oder verzweigtes Alkyl oder Alkenyl mit jeweils bis zu 19 Kohlenstoffatomen bedeutet, die gegebenenfalls durch eine Gruppe der Formel $-OR^{23}$ substituiert sind,
worin

$R^{23}$ Wasserstoff, Benzyl, Triphenylmethyl oder geradkettiges oder verzweigtes Acyl mit bis zu 4 Kohlenstoffatomen bedeutet,

gegebenenfalls in einer isomeren Fom und deren Salze.

**4.** Pyridazino-, Pyrimido-, Pyrazino- und Triazino-indole nach Anspruch 1 bis 3 als Arzneimittel.

**5.** Verfahren zur Herstellung von Pyridazino-, Pyrimido-, Pyrazino- und Triazino-indolen nach Anspruch 1 bis 3, dadurch gekennzeichnet, daß man racemische oder auch bereits enantiomerenreine Carbonsäuren oder deren aktivierte Derivate der allgemeinen Formel (II)

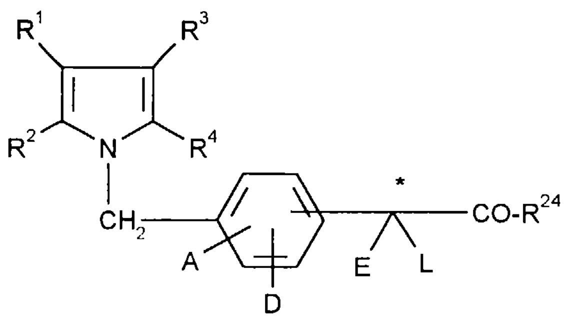

(II)

in welcher

A, D, E, L, $R^1$, $R^2$, $R^3$ und $R^4$ die oben angegebene Bedeutung haben,

und

$R^{24}$ für Hydroxy oder für einen aktivierenden Rest, vorzugsweise Chlorid steht,

mit Verbindungen der allgemeinen Formel (III)

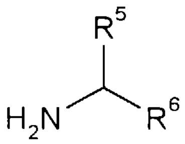

(III)

in welcher

$R^5$ und $R^6$ die oben angegebene Bedeutung haben,

in inerten Lösemitteln, gegebenenfalls in Anwesenheit von Basen und/oder Hilfsstoffen amidiert.

**6.** Arzneimittel enthaltend mindestens ein Pyridazino-, Pyrimido-, Pyrazino -oder Triazino-indol nach Anspruch 1 bis 3 sowie pharmakologisch unbedenkliche Formulierungshilfsmittel.

**7.** Arzneimittel nach Anspruch 6 zur Behandlung von Atherosklerose.

**8.** Verwendung von Pyridazino-, Pyrimido-, Pyrazino- und Triazino-indolen nach Anspruch 1 bis 3 zur Herstellung von Arzneimitteln.

**9.** Verwendung nach Anspruch 8 zur Herstellung von antiatherosklerotisch wirksamen Arzneimitteln.

**10.** Verwendung von Pyridazino-, Pyrimido-, Pyrazino- und Triazino-indolen nach Anspruch 1 bis 3 zur Verminderung oder vollständigen Inhibisierung der Bildung und/oder Freisetzung von Apo B-100-assoziierten Lipoproteinen.